# EUROPEAN PATENT APPLICATION

(11) **EP 4 818 321 A1**
(43) Date of publication of application: **07.10.2026**
(21) Application number: 25167486.7
(22) Date of filing: 31.03.2025
(51) Int. Cl.: C12N 15/113, C12N 15/90

(54) **MODIFIED CHO CELLS WITH A GENETICALLY ENGINEERED FASTER GROWTH RATE**

(71) Applicant: Sartorius Stedim Cellca GmbH, 89081 Ulm (DE)
(72) Inventor: Marzluf, Jannis, 89081 Ulm (DE); Leroux, Ann-Cathrin, 89081 Ulm (DE); Zehe, Christoph, 89081 Ulm (DE); Reif, Oscar-Werner, 89081 Ulm (DE)
(74) Representative: Eisenführ Speiser

(57) **Abstract**

The invention relates to a modified Chinese Hamster Ovary (CHO) cell with an increased growth rate, wherein the cell comprises at least one modification in at least one endogenous coding sequence, selected from a group of target genes, and at least one recombinant protein and/or at least one recombinant RNA. The invention further relates to method for the production of at least one modified CHO cell and a method of production of at least one recombinant protein and/or recombinant RNA. Additionally, the invention relates to the use of at least one modified CHO cell for the production of at least one pharmaceutical drug. The invention also relates to a kit comprising at least one modified CHO cell.

## Description

### Technical Field

The invention relates to a modified Chinese Hamster Ovary (CHO) cell with an increased growth rate, wherein the cell comprises at least one modification in at least one endogenous coding sequence, selected from a group of target genes, and at least one recombinant protein and/or at least one recombinant RNA. The invention further relates to method for the production of at least one modified CHO cell and a method of production of at least one recombinant protein and/or recombinant RNA. Additionally, the invention relates to the use of at least one modified CHO cell for the production of at least one pharmaceutical drug. The invention also relates to a kit comprising at least one modified CHO cell.

### Background of the Invention

Chinese Hamster Ovary (CHO) cells remain the primary workhorse in the biopharmaceutical industry for producing therapeutic proteins, including monoclonal antibodies. CHO cells are the primary production platform for the biopharmaceutical industry, e.g., for nearly 70% of biologics and the vast majority of monoclonal antibodies. (Lalonde & Durocher, Therapeutic glycoprotein production in mammalian cells, J Biotechnol (2017), 251, 128-140). Their unparalleled ability to support complex post-translational modifications, including human-like glycosylation, makes them the preferred choice for therapeutic protein manufacturing.

However, their adaptation to large-scale industrial use has been an ongoing challenge due to their evolutionary origins. Naturally, CHO cells evolved as adherent cells within a multicellular environment rather than as high-yielding suspension cultures in bioreactors. This fundamental mismatch between their biological characteristics and the demands of industrial-scale bioproduction has necessitated extensive genetic modifications to enhance their efficiency. Furthermore, despite their well-established role, the pace of CHO-based cell line development (CLD) is often hampered by relatively slow growth rates, generally ranging from 18 to 22 hours per doubling. This limitation becomes especially problematic when exploring novel biomolecules or scaling up production to meet market demands.

One of the major challenges in engineering CHO cells lies in balancing productivity with cell viability. While genetic modifications aim to increase recombinant protein expression, they often impose metabolic stress that compromises growth and stability. Understanding gene essentiality is another critical aspect, as genome reduction strategies require precise knowledge of which genes can be safely removed without impairing cellular function in a production setting. At the same time, modifications must not negatively impact product quality, particularly in terms of glycosylation patterns, protein folding, and post-translational modifications that are crucial for therapeutic efficacy. Additionally, CHO cells must be adapted to thrive under the dynamic and often stressful conditions of large-scale bioprocessing, where factors such as shear stress, nutrient availability, and high-density culture conditions can affect their performance.

Notably, a single protein often serves multiple functional roles by interacting with myriad partners, forming large complexes, and triggering downstream or feedback effects in various signalling pathways.

Cells synthesize thousands of proteins that converge into interdependent networks. Within these networks, the function of any given protein is shaped not only by its amino acid sequence and three-dimensional structure, but also by its interaction partners and the temporal or spatial context in which it is expressed. For example, multiple proteins assemble into complexes that maintain structural integrity, regulate metabolism, or coordinate signal transduction. When one protein in such a complex is modified or removed, it can have a cascading influence on the composition of the entire complex or on the expression of other proteins that share the same pathway.

Additionally, many proteins act as regulatory hubs: a single protein might influence multiple downstream targets through phosphorylation, ubiquitination, or other post-translational modifications. As a result, even minor perturbations can ripple through an entire pathway or across several pathways (Bonetta L, Protein-protein interactions: interactome under construction, Nature (2010), 486, 851-854).

Despite decades of genetic engineering efforts (Kim at al., CHO cells in biotechnology for production of recombinant proteins: current state and further potential. Appl Microbiol Biotechnol (2012), 93, 917-30), optimizing CHO cells remains a complex challenge that requires a deeper understanding of their biology and a more refined approach to genetic modifications.

WO 2017/051347 A2 relates to the inhibition of cell growth in high-density cell cultures, such as fed-batch cultures designed for the production of recombinant proteins, due to the accumulation of specific metabolites in the culture medium. It is disclosed that the accumulation of metabolites, including 3-(4-hydroxyphenyl)lactate, 4-hydroxyphenylpyruvate, phenyllactate, phenylpyruvate, indolelactate, indolecarboxylic acid, homocysteine, 2-hydroxybutyric acid, isovalerate, 2-methyl butyrate, isobutyrate, and formate, can inhibit cell growth. To mitigate this inhibitory effect, WO 2017/051347 A2 follows an approach in which the synthesis of these inhibitory metabolites is reduced through genetic modification of a confined set of genes, namely Bcat1, Bcat2, Auh, Mecr, Mccc2, Ivd, Hpd, Hgd, Pah, PCBD1, and QDPR.

However, WO 2017/051347 A2 remains limited in scope, as it exclusively focuses on reducing the accumulation of small set of metabolites through targeted genetic modifications, without leveraging broader genomic strategies. The approach described in the prior art represents a conventional and incremental improvement, rather than a utilization of the genetic potential of the cell. This established approach offers only limited benefits, as it does not leverage genome-wide optimizations that could lead to more robust and scalable solutions for high-density cell culture systems.

WO 2009/086215 A2 relates to the genetic engineering of cell lines to achieve improved cell culture phenotypes by modulating the expression of specific genes or proteins. It discloses a population of engineered cells, each containing an engineered construct that up-regulates or down-regulates a defined set of genes to improve certain cell culture characteristics. However, WO 2009/086215 A2 has decisive limitations that stem from the technological constraints of its time. The approach relies on microarrays for gene expression analysis, which, at the time of publication, was a labour-intensive and low-throughput method prone to significant background noise and hybridization artefacts.

Microarray-based techniques often suffer from limited dynamic range, cross-hybridization errors, and difficulties in detecting low-abundance transcripts, leading to unreliable gene expression data. As a result, identifying relevant gene targets was inherently error-prone. Thus, WO 2009/086215 A2 employs a conventional methodology by primarily inferring phenotypic changes in cells through overexpression of selected target genes. Such an approach inherently cannot fully account for the intricacy of gene regulatory networks and cellular feedback loops, and consequently may induce artificial cellular states that diverge from naturally optimized biological functions, yielding suboptimal phenotypic outcomes. Furthermore, it does not incorporate a comprehensive, genome-wide perspective, thus limiting both the range of targeted genes and the potential for more robust optimization.

In addition to growth-related challenges, modern large-scale genomic modifications in CHO cells present significant hurdles. CRISPR-based screens have proven valuable in uncovering gene functions across mammalian cells, often relying on lentiviral (LV) delivery methods. LV has its own challenges. Besides the complexities of virus-handling - including safety concerns and specialized facility requirements - a significant likelihood of multi-copy insertions, which complicates data interpretation and undermines the precision of gene knockouts.

**Xiong et al.** aimed to demonstrate that lentivirus-free single guide RNA (sgRNA) delivery with transient Cas9 expression could minimise the challenges of LV (Xiong et al., An optimized genome-wide, virus-free CRISPR screen for mammalian cells, Cell Reports Methods (2021), 1, 1000622021). Therefore, they employed a landing pad strategy to ensure that each guide RNA cassette was inserted at a single, pre-defined genomic location, to minimize clonal variation caused by random integration events. However, this precondition reflects a strong disadvantage of this virus-free system because it requires the prior engineering of cells with a recombinase landing pad at a defined genomic locus. Establishing this landing pad not only adds time and complexity but also restricts screening to cell lines that are amenable to extensive genetic manipulation. Furthermore, only around 55% editing efficiency was achieved, used approximately 25% fewer sgRNAs than a fully comprehensive library, and encountered about 40% single-copy integration rates.

Overall, there are still several technical hurdles associated with genome-wide screening methodologies - including suboptimal control of integration copy number, potential off-target effects, difficulties with large-scale transfection, and the potential for founder effects due to restriction to the specific, homogeneous, potentially biased phenotype of a single cell clone.

It was thus an object of the present invention to address the great need in the art to identify and establish more efficient and precise methods to speed-up said cell line developmental process to reliably identify genetic targets that improve CHO cell growth.

### Summary of the Invention

The above-identified objects are solved by the technical teaching as provided with the present invention.

In a first aspect there is provided a modified Chinese Hamster Ovary (CHO) cell having an increased growth rate, wherein the cell comprises at least one modification in at least one endogenous coding sequence encoding at least one endogenous protein as defined in **Table 1,** preferably as defined in **Table 2,** wherein the at least one endogenous coding sequence is individually selected from the group of target genes as defined in **Table 6,** preferably as defined in **Table 7** or any homologue, orthologue, or paralogue thereof; and wherein the at least one modification leads to the reduced transcription and/or reduced functional expression of said at least one endogenous protein, or of any homologue, orthologue, or paralogue thereof.

One embodiment relates to the modified CHO cell according to the first aspect, wherein the at least one modification of at least one endogenous coding sequence is combined with at least one modification of at least one different endogenous coding sequence of a target gene selected from the group of HMGB1, STUB1, CAPN1, FOXO3, IFI16, KLHL22, LRRK2, PSAP, DCP1B, DDIT4, ARID1B, BRCC3, CCNG2, LATS2, NPM2, PML, SMARCD1, SWI5, GATA1, PSMD2, HORMAD2, PKP4, VEGFC, NDRG1, ANGPT2, BCAS3, MIA3, SPARC, MACF1, HLX, RALGAPA1, ARHGAP5, KIDINS220, CDH1, RILPL1, RIMS2, STK25, SLC12A2, ADAMTS15, MAPK11, PTBP1, SMAD1, ZEB1, CD24, CHD7, EXT1, JADE3, TF, EBF1, MED11, RXRA, TCOF1, FGF12, NF2, NCOA7, AKAP3, ME2, MPST, NADK2, PCK2, GPC3, PTPRM, GRAP, MBD1, RXRB, CASP9, CYP2C8, RPS19, HOXB3, NDRG1, FAH, LRTOMT, CDS1, OSBPL8, CEMIP, GLB1L2, CRTC3, SLC25A25, ALDH8A1, MID1IP1, HNF1A, OSTC, PANK4, GGCX, GTF2H1, SET, BACH2, ENTPD7, FCER1G, ESX1, MXD3, TBX15, GRIA2, AREL1, FBXO30, TRIM2, TLE1, TRIM2, UBE2Q1, RPL5, RPL7, RPS11, RPL23A, RPL35, SH3BGRL, SLC36A4, XPR1, ZDHHC20, MAPKBP1, TRAF7, EIF3E, BLOC1S6, CADPS, SYTL1, TPRG1L, SSH3, DISP1, GRK6, LZTR1, PAG1, SCN1A, FXYD7, TSPAN32, MAPKBP1, MAPKBP1, MAPKBP1, or of any homologue, orthologue, or paralogue thereof, preferably of a group of target genes from an individual panel as defined in **Table 3;** and the at least one combination of modifications leads to the reduced transcription and/or reduced functional expression of said at least one endogenous protein, or of any homologue, orthologue, or paralogue thereof.

One embodiment relates to the modified CHO cell according to the first aspect or the embodiment thereof, wherein said at least one endogenous protein has an amino acid sequence corresponding to SEQ ID NO: 1 to 235, or any homologue, orthologue, or paralogue thereof having an amino acid sequence having at least 75%, 76%, 77%, 78%, 79%, 80%, 81%, 82%; 83%, 84%, 85%, 86%, 87%, 88%, 89%, 90%, 91%, 92%, 93%, 94%, 95%, 96%, 97%, 98% or 99% sequence identity to a sequence corresponding to SEQ ID NO: 1 to 235, respectively.

One embodiment relates to the modified CHO cell according to the first aspect or any embodiment thereof, wherein the total concentration of the at least one modified endogenous protein is individually reduced by at least 20%, 25%, 30%, 35%, 40%, 45%, 50%, more preferably at least 55%, 60%, 65%, 70%, 75%, 80%, most preferably at least 80%, 81%, 82%, 83%, 84%, 85%, 86%, 87%, 88%, 89%, 90%, 91%, 92%, 93%, 94%, 95%, 96%, 97%, 98%, 99%, 100%, or even 100%, as determined by measuring the relative concentration of the at least one modified endogenous protein in ng/mL per sample obtained in a modified CHO cell in comparison to a reference sample obtained from a CHO wild-type cell not carrying the at least one modification in its genome.

One embodiment relates to the modified CHO cell according to the first aspect or any embodiment thereof, wherein said endogenous protein has a reduced transcription and/or reduced functional expression, wherein The modified CHO cell comprises at least one modification in at least three, at least four, at least five, at least six, at least seven, at least eight, at least nine, at least ten, at least eleven, at least twelve, at least thirteen, at least fourteen, at least fifteen, at least sixteen, at least seventeen, at least eighteen, at least nineteen, at least twenty or more different endogenous coding sequences encoding at least one endogenous protein and at least one recombinant sequence encoding at least one recombinant protein and/or at least one recombinant RNA.

One embodiment relates to the modified CHO cell according to the first aspect or any embodiment thereof, wherein said CHO cell comprises at least one recombinant gene encoding at least one recombinant protein and/or encoding at least one recombinant RNA molecule.

One embodiment relates to the modified CHO cell according to the embodiment before the last, wherein said at least one recombinant protein is a therapeutic molecule.

One embodiment relates to the modified CHO cell according to the first aspect or any embodiment thereof, wherein said at least one modification is selected from the group consisting of at least one insertion, at least one deletions, and at least one substitution, including a base edit, or any combination thereof, preferably wherein said at least one modification is present in exon 1 of the respective endogenous coding sequence, and/or wherein said at least one modification in said endogenous sequence is a frame-shift mutation or a point mutation, the point mutation resulting in a stop codon.

One embodiment relates to the modified CHO cell according to the first aspect or any embodiment thereof, wherein said at least one modification in said at least one endogenous coding sequence allows for an optimized production of the at least one recombinant protein and/or at least one recombinant RNA, (i) wherein said optimized production of the at least one recombinant protein and/or the at least one recombinant RNA is characterized by an increase (a) in the yield of the at least one recombinant protein, and/or (b)in the yield of the at least one RNA encoding said at least one recombinant protein cell, and/or (c) in the yield of the at least one recombinant RNA, in comparison to a reference sample obtained from a CHO wild-type cell not carrying the at least one modification in its genome, wherein the total concentration of the at least one modified endogenous protein is reduced by at least 20%, 25%, 30%, 35%, 40%, 45%, 50%, more preferably at least 55%, 60%, 65%, 70%, 75%, 80%, most preferably at least 80%, 81%, 82%, 83%, 84%, 85%, 86%, 87%, 88%, 89%, 90%, 91%, 92%, 93%, 94%, 95%, 96%, 97%, 98%, 99%, 100%, or even 100%, as determined by measuring the relative concentration of the at least one recombinant protein and/or at least one recombinant RNA in ng/mL per sample obtained in a modified CHO cell in comparison to a reference sample obtained from a CHO wild-type cell not carrying the at least one modification in its genome.

One embodiment relates to the modified CHO cell according to the first aspect or any embodiment thereof, wherein all alleles of said at least one endogenous coding sequence and/or regulatory sequence comprise at least one or more of said at least one modification.

One embodiment relates to the modified CHO cell according to the first aspect or any embodiment thereof, wherein The modified CHO cell comprises at least one modification in at least three, at least four, at least five, at least six, at least seven, at least eight, at least nine, at least ten, at least eleven, at least twelve, at least thirteen, at least fourteen, at least fifteen, at least sixteen, at least seventeen, at least eighteen, at least nineteen, at least twenty or more different endogenous coding sequences encoding at least one endogenous protein and at least one recombinant sequence encoding at least one recombinant protein and/or at least one recombinant RNA, wherein: each endogenous coding sequence is selected from the group consisting of the following KEGG pathways: ribosome, spliceosome, cell cycle, nucleocytoplasmic transport, oxidative phosphorylation, biosynthesis of cofactors, mRNA surveillance pathway, nucleotide excision repair, RNA degradation, proteasome, DNA replication, basal transcription factors, RNA polymerase, homologous recombination and mismatch repair.

One embodiment relates to the modified CHO cell according to the first aspect or any embodiment thereof, wherein the CHO cell undergoes a measurable and quantifiable enhancement of cell size (volume or mass) beyond that observed in a suitable reference or control population, preferably derived by using peak fold-change values to predict growth rate improvements.

A second aspect relates to a method for the production of at least one modified CHO cell according to the first aspect or any embodiment thereof, comprising the steps: (i) providing at least one CHO cell comprising at least one endogenous target nucleic acid segment; (ii) providing at least one genome or transcriptome editing agent comprising at least one RNAi agent, or at least one site-directed endonuclease, preferably being selected from a meganuclease, a ZFN, a TALEN, a CRISPR-nuclease, or a nickase or nuclease-dead variant therefrom, or a nucleic acid molecule encoding the same, and optionally in case of a CRISPR-nuclease: providing at least one suitable, functional guide RNA molecule, or a nucleic acid molecule encoding the same; (iii) introducing into said at least one CHO cell the at least one genome editing agent of step (ii); (iv) obtaining at least one modified CHO cell comprising at least one modification in said at least one endogenous target nucleic acid segment according to step (i); (v) optionally: selecting a further target nucleic acid segment and repeating steps (i) to (iv) at least one time to obtain at least one modified CHO cell comprising at least one further modification in a further endogenous target nucleic acid segment.

One embodiment relates to the method according to the second aspect, wherein (i) in step (ii) of the second aspect a CRISPR-nuclease is used; and (ii) optionally: wherein a Cas enzyme is constitutively expressed by said at least one modified CHO cell; and (iii) wherein the at least one suitable functional guide RNA molecule comprises a sequence corresponding to any of the nucleic acid sequences according to SEQ ID NOs: 236 to 1636 or corresponding to a nucleic acid sequence having at least 75%, 76%, 77%, 78%, 79%, 80%, 81%, 82%; 83%, 84%, 85%, 86%, 87%, 88%, 89%, 90%, 91%, 92%, 93%, 94%, 95%, 96%, 97%, 98% or 99% sequence identity to any one of the sequences according to SEQ ID NOs: 236 to 1636.

A third aspect relates to a method for the production of at least one recombinant molecule, preferably at least one recombinant protein and/or recombinant RNA, comprising the steps: (a) providing at least one modified CHO cell according to the first aspect or any embodiment thereof, wherein the at least one modified CHO cell comprises at least one recombinant gene encoding at least one recombinant protein, DNA or RNA; (b) culturing said at least one target cell in a culture medium such that at least one recombinant molecule is transcribed and/or translated; (c) harvesting said at least one recombinant molecule; (d) purifying and/ or decontaminating said at least one recombinant molecule, preferably the at least one recombinant protein.

A fourth aspect relates to the use of at least one modified CHO cell according to the first aspect or any embodiment thereof for the production of at least one pharmaceutical drug.

A fifth aspect relates to a kit comprising (a) at least one modified CHO cell according to the first aspect or any embodiment thereof, the modified CHO cell or the kit comprising: (b.i)at least one nucleic acid molecule suitable for expressing at least one recombinant molecule of interest, preferably at least one recombinant protein of interest; optionally means for introducing the same into the modified CHO cell genome, or alternatively (b.ii) alternatively at least one nucleic acid molecule encoding at least one recombinant molecule, preferably at least one recombinant protein; optionally means for introducing the same into the modified CHO cell genome; or (b.iii) alternatively: at least one nucleic acid molecule suitable for transcribing at least one recombinant RNA molecule; optionally means for introducing the same into the modified CHO cell genome; or (b.iv) alternatively: at least one nucleic acid molecule encoding at least one recombinant RNA molecule; optionally means for introducing the same into the modified CHO cell genome; and (c) optionally: culture medium suitable for the at least one modified CHO cell according to step (a); and (i) to replicate the at least one nucleic acid molecule according to any of the steps (b.i), (b.ii), (b.iii), and (b.iv) endogenously or exogenously; and (ii.a) to express the at least one recombinant according to step (b.i) and/or (b.ii), and/or to transcribe the at least one recombinant RNA molecule according to step (b.iii) and/or (b.iv).

### Brief Description of Figures

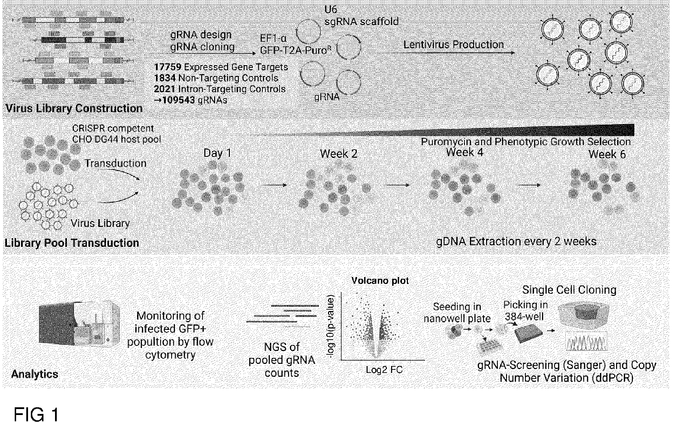

**FIG 1****: Workflow for the generation of pooled CHO KO Minilibrary**
   A previously validated CRISPR competent Cas-expressing cell pool was infected with a whole genome covering lentiviral library. Shaking-based transduction at a target MOI of 0.15 was performed. Throughout the period of the screen gDNA was extracted bi-weekly to serve as point of comparison against the sgRNA distribution in the plasmid library. Additionally, Single cell clones were isolated three weeks post-transduction for the analysis of transduced copy-numbers by Sanger-sequencing and ddPCR. The gDNA samples were used to prepare a Next Generation Sequencing (NGS) library, followed by Illumina HiSeq sequencing to assemble read counts of all sgRNAs. The transduced population was tracked using flow cytometry and the expressed GFP reporter.

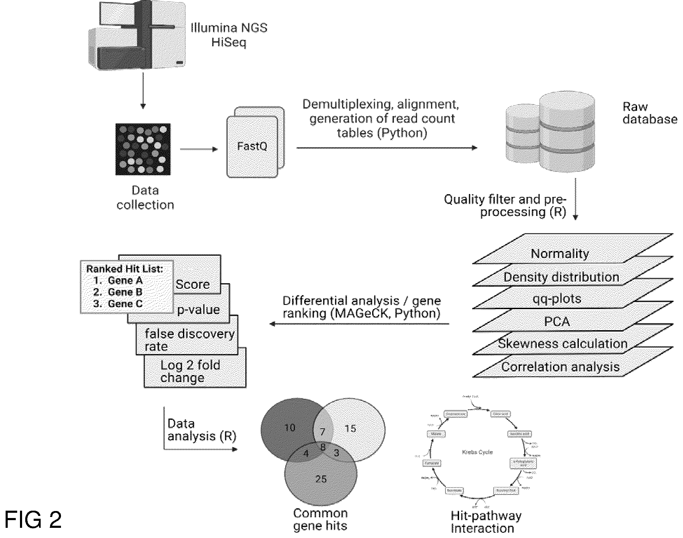

**FIG 2****: Bioinformatics workflow for data analysis**
   Genomic DNA from all time points was used to prepare an NGS library for Illumina HiSeq sequencing. The resulting FastQ files were processed for demultiplexing, alignment, and generation of read count tables. These tables underwent quality filtering and preprocessing for MAGeCK for calculating statistics of changes in read count distributions (Li et al., MAGeCK enables robust identification of essential genes from genome-scale CRISPR/Cas9 knockout screens, *Genome Biology* (2014), 15, 554). Through MAGeCK, comparisons across different time points, cultivation modes, and conditions were performed to identify gene hits in enriched and depleted direction. Data were analyzed, visualized, and annotated using pathway analysis in R to identify common gene hits across comparisons. Genes were further ranked based on their consistency across comparisons, the number of occurrences, and the magnitude of fold changes observed in sgRNA counts.
**FIG 3****: Results from Minilibrary Screen covering 20 Target Genes I**
   A: GFP-positive cells after transduction with MOI of 0.15. B: GFP-positive population throughout the selection period.
**FIG 4****: Results from Minilibrary Screen covering 20 Target Genes II**
   A: Fraction of clones with a unique sgRNA sequence integrated from the sgRNA library determined by Sanger sequencing. B: CNV analysis from ddPCR.
**FIG 5****: Results from Minilibrary Screen covering 20 Target Genes III**
   The α-mean log2 fold change is plotted against α-RRA. This comparison involves time point replicates against the plasmid library. The intron-targeting sgRNA groups, non-targeting sgRNA groups, and significantly enriched or depleted genes are depicted by the symbols according to the figure legend. The cutoff criteria include a false discovery rate (fdr) < 0.05 or α-mean log2 fold change > 1 and p-value < 0.025 for positive enrichment, and fdr < 0.05 or α-mean log2 fold change < 0 and p-value < 0.025 for negative selection.

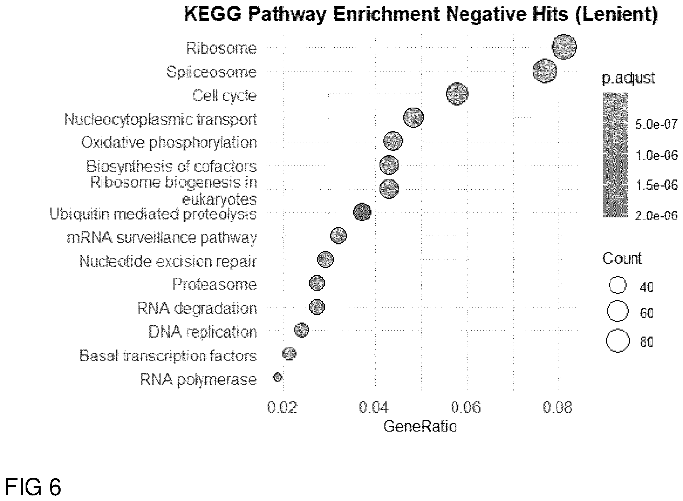

**FIG 6****: Results from Minilibrary Screen covering 20 Target Genes IV**
   KEGG pathway analysis of deleted genes. Significantly enriched pathways in negative hits are shown.

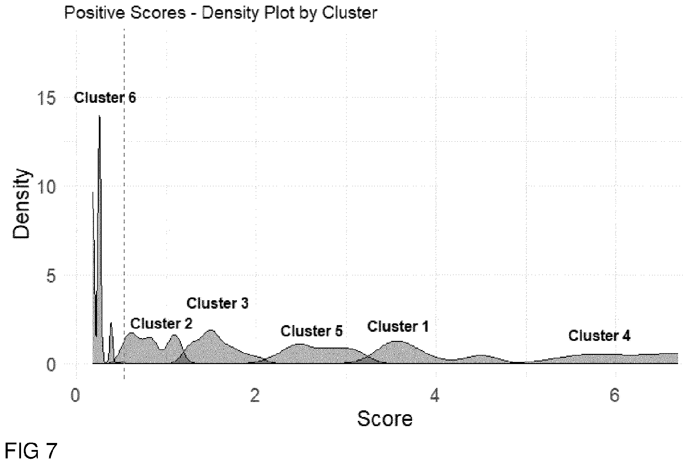

**FIG 7****: Clustering analysis of positive gene hits to derive final gene list I**
   Scores of genes across different clusters assigned by k means clustering. Cutoff marked between the lowest scoring cluster and the second lowest (dotted vertical line).

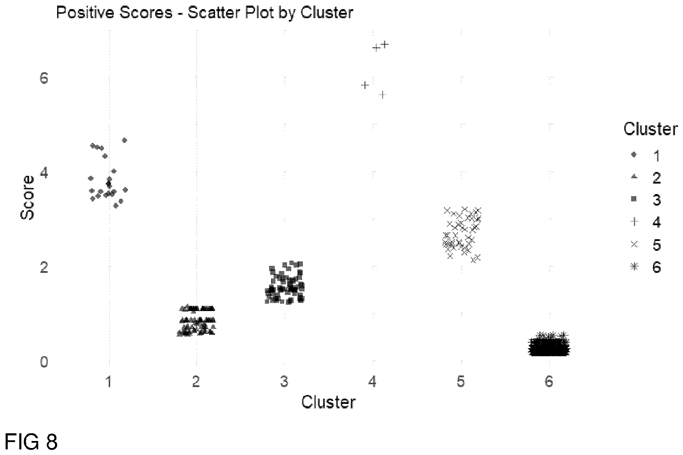

**FIG 8****: Clustering analysis of positive gene hits to derive final gene list II**
   Score distribution inside of all Clusters. The different clusters are depicted by the symbols according to the figure legend.

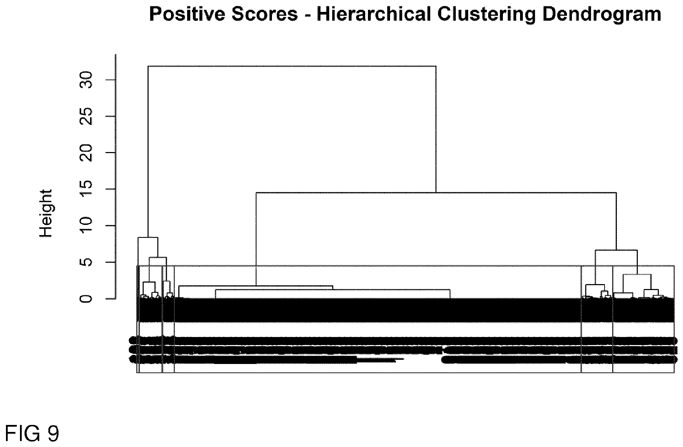

**FIG 9****: Clustering analysis of positive gene hits to derive final gene list III**
   Hierarchical clustering to derive the ideal number of clusters for k-means clustering analysis.

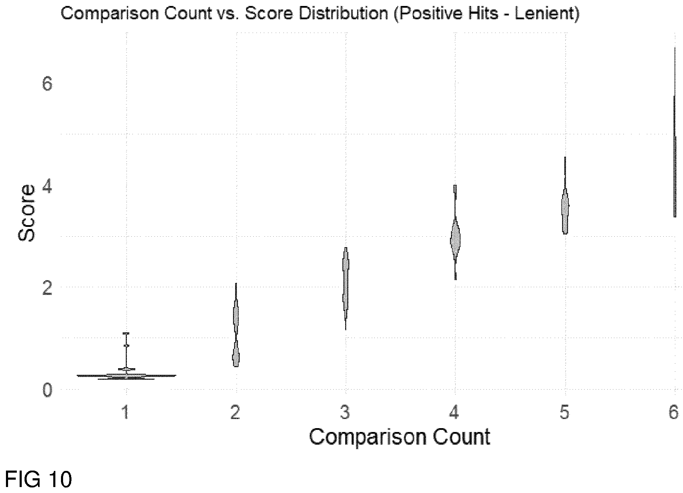

**FIG 10****: Clustering analysis of positive gene hits to derive final gene list IV**
   Scores of individual genes plotted against the comparison count in terms of the comparisons in which the gene was significantly positively enriched using custom cutoff criteria described earlier.

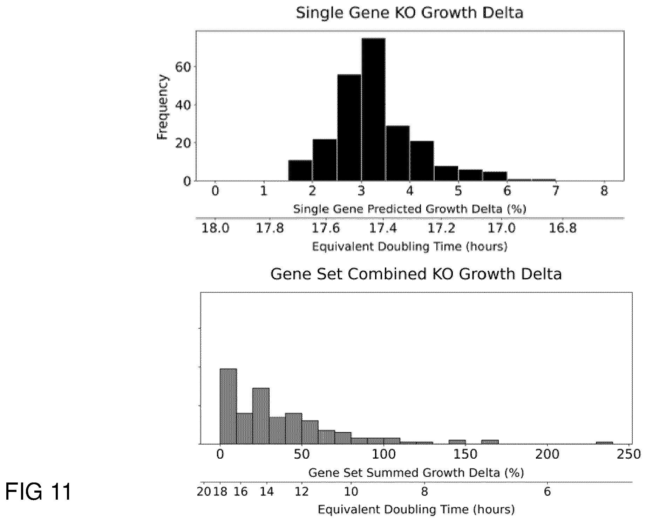

FIG 11: **Predicted growth improvements for CHO cells following gene knockout**
   Left histogram: predicted growth rate improvements for individual gene knockouts. Right histogram: cumulative predicted improvements for functionally grouped gene sets. The lower secondary x-axis translates percentage improvements into equivalent cellular doubling times, assuming an 18-hour baseline doubling time.

### Brief Description of Sequences

**Table 1**

| **SEQ ID NO** | **Endogenous Protein** | **SEQ ID NO** | **Endogenous Protein** | **SEQ ID NO** | **Endogenous Protein** |
|---|---|---|---|---|---|
| 1 | PARD3B | 79 | LATS2 | 157 | PAG1 |
| 2 | GRAP | 80 | APPBP2 | 158 | TENM4 |
| 3 | ZDHHC20 | 81 | BCAS3 | 159 | CCDC34 |
| 4 | CLEC4F | 82 | UNC5B | 160 | EIF1 |
| 5 | PHF7 | 83 | GRK6 | 161 | SYTL1 |
| 6 | CRLF2 | 84 | MXD3 | 162 | CEMIP |
| 7 | IGH-VJ558 | 85 | SIMC1 | 163 | ZNF764 |
| 8 | ZNF420 (fragment) | 86 | APOPT1 | 164 | TCN2 |
| 9 | Zinc finger protein | 87 | SLC36A2 | 165 | MORC4 |
| 10 | RHOXF2B | 88 | SPARC | 166 | GTF2H1 |
| 11 | FXYD7 | 89 | IFI204 | 167 | PSMD2 |
| 12 | CCDC106 | 90 | DCP1B | 168 | SSH1 |
| 13 | ZNF420 | 91 | N-acetylglucosaminyl-proteoglycan 4-beta-glucuronosyltransferase | 169 | AREL1 |
| 14 | PRRT3 | 92 | EIF3E | 170 | XPNPEP1 |
| 15 | AKAP3 | 93 | ANGPT2 | 171 | SWI5 |
| 16 | ZNF865 | 94 | SCN1A | 172 | LHX6 |
| 17 | FCGBP | 95 | STUB1 | 173 | ENTPD7 |
| 18 | CLEC2H | 96 | Hydroxyacylglutathione hydrolase-like protein | 174 | STK17B |
| 19 | SLC25A25 | 97 | UBE2Q1 | 175 | zinc finger protein |
| 20 | MIA3 | 98 | SRD5A2 | 176 | OSTC |
| 21 | FCER1G | 99 | DPP9 | 177 | LRRK2 |
| 22 | SPATA31A6 | 100 | SNX25 | 178 | RXRB |
| 23 | Domain of unknown function | 101 | phosphoenolpyruvate carboxykinase (GTP) | 179 | SP110 |
| 24 | TMEM55A | 102 | ASB15 | 180 | FAM172A |
| 25 | SULT3A1 | 103 | H2AFJ | 181 | CYP2C18 |
| 26 | LRRC19 | 104 | Reverse transcriptase | 182 | TBX18 |
| 27 | 2-aminomuconic semialdehyde dehydrogenase | 105 | PML | 183 | PANK4 |
| 28 | AHDC1 | 106 | KLHL22 | 184 | TCEB2 |
| 29 | Nuclear receptor coactivator | 107 | PTBP1 | 185 | TSKS |
| 30 | SLC12A2 | 108 | TMEM79 | 186 | BCL2L12 |
| 31 | MBD1 | 109 | VEGFC | 187 | MPST |
| 32 | TNS2 | 110 | RNA-binding protein 26 | 188 | TOMT |
| 33 | CHD7 | 111 | PKP4 | 189 | HMGB1 |
| 34 | MUC19 | 112 | FAM98B | 190 | RPL7 |
| 35 | SETBP1 | 113 | SLC7A14 | 191 | OR5112 |
| 36 | METAP1 | 114 | NFS1 | 192 | ARL4D |
| 37 | CHIC2 | 115 | TARS | 193 | NWD2 |
| 38 | T-cell receptor | 116 | RPS19 | 194 | Immunoglobulin V-Type |
| 39 | SERPINI2 | 117 | PDE1C | 195 | VMN2R116 |
| 40 | LMAN2L | 118 | NADK2 | 196 | BRD9 |
| 41 | CCNG2 | 119 | SET | 197 | CAPN1 |
| 42 | CD24 | 120 | SET | 198 | PTAR1 |
| 43 | DISP1 | 121 | CDH1 | 199 | NDRG4 |
| 44 | HLX | 122 | RPS11 | 200 | SMAD1 |
| 45 | TRHR | 123 | WNT1-inducible-signaling pathway protein 2 | 201 | FAM83A |
| 46 | MID1IP1 | 124 | KIDINS220 | 202 | BACH2 |
| 47 | MAP3K7CL | 125 | BLOC1S6 | 203 | hypothetical protein |
| 48 | FGF12 | 126 | ZEB2 | 204 | CRTC2 |
| 49 | Eukaryotic protein of unknown function | 127 | MACF1 | 205 | hypothetical protein |
| 50 | GLB1L2 | 128 | MSANTD4 | 206 | hypothetical protein |
| 51 | ADAMTS15 | 129 | Vitamin K-dependent gamma-carboxylase | 207 | TECPR2 |
| 52 | ZNF462 | 130 | RPL23A | 208 | hypothetical protein |
| 53 | KLHL23 | 131 | DEPTOR | 209 | hypothetical protein |
| 54 | NPM2 | 132 | OLFR1013 | 210 | hypothetical protein |
| 55 | LRRC48 | 133 | TCOF1 | 211 | ENV |
| 56 | TUT4 | 134 | Oxysterol-binding protein-related protein | 212 | PNPLA4 |
| 57 | MED11 | 135 | ME2 | 213 | hypothetical protein |
| 58 | CHRM3 | 136 | SLC24A1 | 214 | Reverse transcriptase |
| 59 | TCP11L1 | 137 | HOXC6 | 215 | FAH |
| 60 | NTSR2 | 138 | RPL35 | 216 | FOXO3 |
| 61 | TF | 139 | RIMS1 | 217 | CTNNA3 |
| 62 | TPRG1L | 140 | DNAJB14 | 218 | KDM5A |
| 63 | WDR62 | 141 | MORF4L2 | 219 | ARID1B |
| 64 | CNNM3 | 142 | FBXO30 | 220 | LZTR1 |
| 65 | TLE3 | 143 | TSPAN32 | 221 | GPC3 |
| 66 | JADE3 | 144 | PCDHGC5 | 222 | TRAF7 |
| 67 | STK25 | 145 | CCDC32 | 223 | HNF1A |
| 68 | UNC80 | 146 | BRCC3 | 224 | PTPRM |
| 69 | MLLT6 | 147 | RPL35 | 225 | CTNNA1 |
| 70 | TRIM2 | 148 | OR7G1 | 226 | GATA1 |
| 71 | HOXB3 | 149 | SH3BGRL | 227 | RPL5 |
| 72 | SLC36A4 | 150 | COL4A2 | 228 | NF2 |
| 73 | UBALD1 | 151 | PSAP | 229 | RPL5 |
| 74 | Adipocyte plasma membrane-associated protein | 152 | DDIT4 | 230 | SMARCD1 |
| 75 | uncharacterized protein | 153 | Mitogen-activated protein kinase 11 | 231 | EBF2 |
| 76 | XPR1 | 154 | ST5 | 232 | CDS1 |
| 77 | CADPS | 155 | Rap/ran-GAP | 233 | CASP9 |
| 78 | GRIA2 | 156 | RILPL1 | 234 | ARHGAP35 |
| | | | | 235 | RXRA |

**Table 2**

| **SEQ ID NO** | **Endogenous Protein** | **SEQ ID NO** | **Endogenous Protein** | **SEQ ID NO** | **Endogenous Protein** |
|---|---|---|---|---|---|
| 1 | PARD3B | 79 | LATS2 | 157 | PAG1 |
| 2 | GRAP | 80 | APPBP2 | 158 | TENM4 |
| 3 | ZDHHC20 | 81 | BCAS3 | 159 | CCDC34 |
| 4 | CLEC4F | 82 | UNC5B | 160 | EIF1 |
| 5 | PHF7 | 83 | GRK6 | 161 | SYTL1 |
| 6 | CRLF2 | | | 162 | CEMIP |
| 7 | IGH-VJ558 | 85 | SIMC1 | 163 | ZNF764 |
| 8 | ZNF420 (fragment) | 86 | APOPT1 | 164 | TCN2 |
| 9 | Zinc finger protein | 87 | SLC36A2 | 165 | MORC4 |
| 10 | RHOXF2B | 88 | SPARC | 166 | GTF2H1 |
| 11 | FXYD7 | 89 | IFI204 | 167 | PSMD2 |
| 12 | CCDC106 | 90 | DCP1B | 168 | SSH1 |
| 13 | ZNF420 | 91 | N-acetylglucosaminyl-proteoglycan 4-beta-glucuronosyltransferase | 169 | AREL1 |
| 14 | PRRT3 | 92 | EIF3E | | |
| 15 | AKAP3 | 93 | ANGPT2 | 171 | SWI5 |
| 16 | ZNF865 | 94 | SCN1A | 172 | LHX6 |
| 17 | FCGBP | 95 | STUB1 | 173 | ENTPD7 |
| 18 | CLEC2H | 96 | Hydroxyacylglutathione hydrolase-like protein | 174 | STK17B |
| 19 | SLC25A25 | 97 | UBE2Q1 | 175 | zinc finger protein |
| 20 | MIA3 | 98 | SRD5A2 | 176 | OSTC |
| 21 | FCER1G | 99 | DPP9 | 177 | LRRK2 |
| 22 | SPATA31A6 | 100 | SNX25 | 178 | RXRB |
| 23 | Domain of unknown function | | | 179 | SP110 |
| 24 | TMEM55A | 102 | ASB15 | 180 | FAM172A |
| 25 | SULT3A1 | 103 | H2AFJ | 181 | CYP2C18 |
| 26 | LRRC19 | 104 | Reverse transcriptase | 182 | TBX18 |
| 27 | 2-aminomuconic semialdehyde dehydrogenase | 105 | PML | 183 | PANK4 |
| 28 | AHDC1 | 106 | KLHL22 | 184 | TCEB2 |
| 29 | Nuclear receptor coactivator | 107 | PTBP1 | 185 | TSKS |
| 30 | SLC12A2 | 108 | TMEM79 | 186 | BCL2L12 |
| 31 | MBD1 | 109 | VEGFC | 187 | MPST |
| 32 | TNS2 | 110 | RNA-binding protein 26 | 188 | TOMT |
| 33 | CHD7 | 111 | PKP4 | 189 | HMGB1 |
| 34 | MUC19 | 112 | FAM98B | 190 | RPL7 |
| 35 | SETBP1 | 113 | SLC7A14 | 191 | OR5112 |
| 36 | METAP1 | 114 | NFS1 | 192 | ARL4D |
| 37 | CHIC2 | 115 | TARS | 193 | NWD2 |
| 38 | T-cell receptor | 116 | RPS19 | 194 | Immunoglobulin V-Type |
| 39 | SERPINI2 | 117 | PDE1C | 195 | VMN2R116 |
| 40 | LMAN2L | 118 | NADK2 | 196 | BRD9 |
| 41 | CCNG2 | 119 | SET | 197 | CAPN1 |
| 42 | CD24 | 120 | SET | 198 | PTAR1 |
| 43 | DISP1 | 121 | CDH1 | 199 | NDRG4 |
| 44 | HLX | 122 | RPS11 | 200 | SMAD1 |
| 45 | TRHR | 123 | WNT1-inducible-signaling pathway protein 2 | 201 | FAM83A |
| 46 | MID1IP1 | 124 | KIDINS220 | 202 | BACH2 |
| 47 | MAP3K7CL | 125 | BLOC1S6 | 203 | hypothetical protein |
| 48 | FGF12 | 126 | ZEB2 | 204 | CRTC2 |
| 49 | Eukaryotic protein of unknown function | 127 | MACF1 | 205 | hypothetical protein |
| 50 | GLB1L2 | 128 | MSANTD4 | 206 | hypothetical protein |
| 51 | ADAMTS15 | 129 | Vitamin K-dependent gamma-carboxylase | 207 | TECPR2 |
| 52 | ZNF462 | 130 | RPL23A | 208 | hypothetical protein |
| 53 | KLHL23 | 131 | DEPTOR | 209 | hypothetical protein |
| 54 | NPM2 | 132 | OLFR1013 | 210 | hypothetical protein |
| 55 | LRRC48 | 133 | TCOF1 | 211 | ENV |
| 56 | TUT4 | 134 | Oxysterol-binding protein-related protein | 212 | PNPLA4 |
| 57 | MED11 | 135 | ME2 | 213 | hypothetical protein |
| 58 | CHRM3 | 136 | SLC24A1 | 214 | Reverse transcriptase |
| 59 | TCP11L1 | 137 | HOXC6 | 215 | FAH |
| 60 | NTSR2 | 138 | RPL35 | 216 | FOXO3 |
| 61 | TF | 139 | RIMS1 | 217 | CTNNA3 |
| 62 | TPRG1L | 140 | DNAJB14 | 218 | KDM5A |
| 63 | WDR62 | 141 | MORF4L2 | 219 | ARID1B |
| 64 | CNNM3 | 142 | FBXO30 | 220 | LZTR1 |
| 65 | TLE3 | 143 | TSPAN32 | 221 | GPC3 |
| 66 | JADE3 | 144 | PCDHGC5 | 222 | TRAF7 |
| | | 145 | CCDC32 | 223 | HNF1A |
| 68 | UNC80 | 146 | BRCC3 | 224 | PTPRM |
| 69 | MLLT6 | 147 | RPL35 | 225 | CTNNA1 |
| 70 | TRIM2 | 148 | OR7G1 | 226 | GATA1 |
| 71 | HOXB3 | 149 | SH3BGRL | 227 | RPL5 |
| 72 | SLC36A4 | 150 | COL4A2 | 228 | NF2 |
| 73 | UBALD1 | 151 | PSAP | 229 | RPL5 |
| 74 | Adipocyte plasma membrane-associated protein | 152 | DDIT4 | 230 | SMARCD1 |
| 75 | uncharacterized protein | 153 | Mitogen-activated protein kinase 11 | 231 | EBF2 |
| 76 | XPR1 | 154 | ST5 | 232 | CDS1 |
| 77 | CADPS | 155 | Rap/ran-GAP | 233 | CASP9 |
| 78 | GRIA2 | 156 | RILPL1 | 234 | ARHGAP35 |
| | | | | 235 | RXRA |

The respective sgRNAs are individually defined by SEQ ID NO: 236 to 1636.

### Definitions

The term "expression" refers to the process of transcription (e.g., of a functional guide RNA) and translation (of a protein or polypeptide) within a host cell. The level or degree of expression of a product gene to the cognate RNA in a host cell can be determined on the basis of either the amount of corresponding mRNA that is present in the cell or the amount of the protein encoded by the product gene that is produced by the cell. For example, mRNA transcribed from a product gene is desirably quantitated by northern hybridization (see, e.g., Sambrook et al., Molecular Cloning: A Laboratory Manual, pp. 7.3- 7.57 (Cold Spring Harbor Laboratory Press, 1989). The polypeptide or protein encoded by a product gene can be quantitated either by assaying for the biological activity of the protein or by using assays that are independent of such activity, such as western blotting using antibodies that are capable of reacting with the protein (see, e.g., Sambrook et al., Molecular Cloning: A Laboratory Manual, pp. 18.1-18.88 (Cold Spring Harbor Laboratory Press, 1989).

"Functional expression" is defined as the successful production of a biologically active protein or gene product in a host cell, ensuring that it maintains its intended structure, function, and activity. This involves proper transcription, translation, folding, and post-translational modifications necessary for the protein to perform a biochemical function. A "reduced functional expression" as used herein in turn refers to the reduction and/or elimination of the expression of one or more endogenous products relative to the expression level of the endogenous product(s) in an unmodified cell. A reduction of functional expression may comprise a reduction and/or complete elimination of the functional endogenous product (on RNA and/or protein level).

"Harvesting" refers to procedure of obtaining a recombinant protein, including a recombinant peptide, of interest, usually at the endpoint of the culture of the host cell. Harvesting can also be done continuously throughout a cultivation process, e.g. in perfusion culture.

A "homologue" refers to a gene, protein, or structure that shares a common evolutionary ancestor with another. Homology is a broad term encompassing both orthologues and paralogues, meaning that two sequences are homologous if they are derived from a common ancestor, regardless of how they have evolved since.

"Increase cell growth" refers to a measurable and quantifiable enhancement of cell size (volume or mass) beyond that observed in a suitable reference or control population. This process typically involves the synthesis and accumulation of cellular components. It primarily occurs during the G1 and G2 phases of the cell cycle. An "increased growth rate" may be preferably derived by using peak fold-change values to predict growth rate improvements, preferably by employing the methodology of Example 3.

"Optimized production" refers to the enhancement of recombinant biomolecule expression, synthesis, and processing through genetic, metabolic, and bioprocess engineering strategies. This optimization aims to maximize yield, stability, and functional integrity while improving resource efficiency, scalability, and reproducibility. It is preferably assessed by measuring the relative concentration of the target molecule (e.g., recombinant protein or RNA) in modified CHO cells compared to a reference CHO cell line under standardized conditions.

"Orthologues" are defined as homologous genes in different species that have evolved from a single ancestral gene through speciation events. They typically retain the same or similar functions across species.

"Paralogues" are defined as homologous genes that arise within the same species due to gene duplication events. Over time, they may evolve new functions (neofunctionalization) or retain redundant roles (subfunctionalization).

A "recombinant molecule" refers to a nucleic acid or protein that has been artificially modified or engineered through molecular biology techniques to contain genetic sequences not naturally found together. This can involve the insertion, deletion, or alteration of genetic material within a host cell or system.

A "recombinant protein", which is a protein expressed from a genetically engineered nucleic acid sequence, such as a therapeutic monoclonal antibody, an enzyme for industrial applications, or a fluorescent protein (e.g., EGFP). The term "recombinant protein" as used herein, refers to any protein, peptide, or fragment thereof that can be produced upon exogenous introduction into a host cell, typically through recombinant (i.e., foreign) DNA or RNA. This may occur via a plasmid, a viral vector, or as a transgene introduced through genome editing or similar methods. Additionally, a "recombinant protein" may also be encoded by an endogenous gene that has been modified using genome editing technologies, including base editing, prime editing, and insertion/deletion (InDel) mutations induced by genome editing. A "recombinant protein" may serve as a therapeutic protein, peptide, or fragment thereof, or it may be any other functional protein, peptide, or fragment. Examples include antibodies, their variants, or functional fragments, as well as any other prophylactic or therapeutic proteins. Furthermore, any artificially introduced or modified protein, such as a fluorescent protein (e.g., EGFP), is also considered a recombinant protein, provided that the protein or its fragment has been inserted, mutated, or otherwise artificially generated within the host cell of interest.

"Recombinant RNA" refers to an RNA molecule transcribed from recombinant DNA or synthetically engineered RNA sequences, including mRNA vaccines, guide RNAs for CRISPR-based genome editing, or RNA therapeutics.

The terms "RNA interference" (RNAi), "RNA silencing," and "gene silencing" are used interchangeably herein to refer to a gene down-regulation (or knockdown) mechanism that has been demonstrated to exist in all eukaryotes. This mechanism was first identified in plants, where it was termed "post-transcriptional gene silencing" (PTGS). In RNAi, small RNA molecules guide specific effector proteins to a target nucleotide sequence through complementary base pairing, leading to the degradation of the target mRNA.

An "RNAi agent" typically consists of sense and antisense sequences, which are complementary to each other and arranged in reverse orientation within a nucleic acid sequence. When a nucleic acid construct contains both sense and corresponding antisense sequences, their transcription results in an RNA double-stranded structure, often forming an RNA hairpin. In an RNA hairpin, the sense and antisense sequences pair to form a double-stranded region, while an intervening intron loop sequence separates them, forming the loop of the hairpin structure. An RNAi agent may also contain multiple sense-antisense pairs, resulting in the formation of multiple hairpin loops.

The term target nucleic acid segment is defined as a nucleotide sequence, typically a DNA sequence, which can be subjected to modification using a site-directed endonuclease.

A "therapeutic molecule" or refers to a recombinant protein as defined above, or any other recombinant nucleic acid molecule that has a prophylactic or curative effect when used to treat a subject in need thereof. Typically, a target nucleic acid segment is part of a genome.

"Upstream processing" (USP) in the context of cell cultures refers to the entirety of cultivation steps taking place prior to the cell harvest.

Whenever the present disclosure relates to the percentage of identity of nucleic acid or amino acid sequences to each other these values define those values as obtained by using the EMBOSS Water Pairwise Sequence Alignments (nucleotide) programme (www.ebi.ac.uk/Tools/psa/emboss_water/) nucleic acids or the EM-BOSS Water Pairwise Sequence Alignments (protein) programme (www.ebi.ac.uk/Tools/psa/emboss_water/) for amino acid sequences. Alignments or sequence comparisons as used herein refer to an alignment over the whole length of two sequences compared to each other. Those tools provided by the European Molecular Biology Laboratory (EMBL) European Bioinformatics Institute (EBI) for local sequence alignments use a modified Smith-Waterman algorithm (see www.ebi.ac.uk/Tools/psa/ and SMITH, T.F. 5 & WATERMAN, M.S. "Identification of common molecular subsequences" Journal of Molecular Biology, 1981 147 (1):195-197). When conducting an alignment, the default parameters defined by the EMBL-EBI are used. Those parameters are (i) for amino acid sequences: Matrix = BLOSUM62, gap open penalty = 10 and gap extend penalty = 0.5 or (ii) for nucleic acid sequences: Matrix = DNAfull, gap open penalty = 10 and gap extend penalty = 0.5. The skilled person is well aware of the fact that, for example, a sequence encoding a protein can be "codon-optimized" if the respective sequence is to be used in another organism in comparison to the original organism a molecule originates from.

### Detailed Description

As specified in the background section, the development of CHO cell lines has been hindered by inefficient gene-editing methodologies, challenges in controlling integration copy number, and difficulties in large-scale transfection. Although CHO cells remain the standard platform for producing complex therapeutic proteins with human-like post-translational modifications, scaling their proliferation without compromising product quality has proven difficult. Rapidly dividing cultures often suffer from metabolic bottlenecks and diminished protein fidelity.

Traditional CHO engineering methods have focused on single genes or small gene clusters, the complex interplay of pathways involved in growth, metabolism, and protein assembly demands a more sophisticated, systems-level approach.

The present invention solves these limitations by providing an advanced CHO cell line development methodology, integrating genomics data and advanced bioengineering methods. This enables by precise, genome-wide modifications a more precise and delicate approach to cell line development. Specifically, these systems-level modifications overcome the long-standing challenge of balancing faster growth with robust biotherapeutic production and establish a new generation of CHO-based manufacturing platforms.

To address the above mentioned need to of a modified CHO cell line with increased growth rate, the present invention thus provides in a first aspect a modified Chinese Hamster Ovary (CHO) cell having an increased growth rate, wherein the cell comprises at least one modification in at least one endogenous coding sequence encoding at least one endogenous protein as defined in **Table 1,** preferably as defined in **Table 2,** wherein the at least one endogenous coding sequence is individually selected from the group of target genes as defined in **Table 6,** preferably as defined in **Table 7** or any homologue, orthologue, or paralogue thereof; and wherein the at least one modification leads to the reduced transcription and/or reduced functional expression of said at least one endogenous protein, or of any homologue, orthologue, or paralogue thereof.

In a preferred embodiment, the productivity of the modified CHO cell is substantially maintained or even increased in comparison to an unmodified CHO reference cell not carrying the at least one modification in the at least one endogenous coding sequence encoding the at least one endogenous protein.

As used herein, the term "productivity" refers to the amount of recombinant protein produced by a modified CHO cell over a given period of time under defined culture conditions relative to an unmodified reference cell line. Productivity may be measured by any methodology routinely used in the art.

Advantageously, even moderate enhancements in growth, when properly matched with stable recombinant molecule expression, can lead to substantial gains in manufacturing capacity, cost efficiency, and ultimate product throughput.

Preferably, productivity may be measured in volumetric terms (e.g., milligrams of protein per liter of culture per day) or specific terms (e.g., picograms of protein per cell per day).

In certain embodiments, the modified CHO cell exhibits a productivity that is substantially the same compared to an unmodified CHO reference cell under comparable cell culture conditions.

In preferred embodiments, the modified CHO cell exhibits a productivity that is significantly increased compared to an unmodified CHO reference cell under comparable cell culture conditions.

In preferred embodiments, the modified CHO cell may exhibit an insignificantly decreased productivity compared to an unmodified CHO reference cell under comparable cell culture conditions.

An insignificantly decreased productivity means any reduction of produced protein amount by the modified CHO cell that does not exceed a statistically insignificant margin of at least 1%, at least 2%, at least 3%, at least 4%, at least 5%, at least 6%, at least 7%, at least 8%, at least 9%, or at least 10%.

A significantly increased productivity means any of produced protein amount by the modified CHO cell that does not exceed a statistically insignificant margin of at least 1%, at least 2%, at least 3%, at least 4%, at least 5%, at least 6%, at least 7%, at least 8%, at least 9%, or at least 10%.

In certain embodiments, the modified CHO cell may be selected from, or may be derived from, meaning originating from, a CHO cell variant selected from the group consisting of CHO-K1, CHO-DXB11 (synonymously referred to as CHO-DUKX), CHO-DG44, CHO-S, and CHO-Pro minus, or a CHO GS knockout. Alternatively, the modified CHO cell according to the present invention may also be derived from other CHO cell variants.

In a preferred embodiment, the modified CHO cell may be, or may be derived from a CHODG44.

In another preferred embodiment, the modified CHO cell may be, or may be derived from a CHO-K1.

In yet a preferred embodiment, the modified CHO cell may be, or may be derived from CHO-DXB11 (synonymously referred to as CHO-DUKX).

In yet another preferred embodiment, the CHO cell may be, or may be derived from a CHO GS knock out cell.

In one preferred embodiment, the modified CHO cell may be derived from CHO-S, or it may be derived from CHO-Pro minus.

**Table 3** lists individual panels of advantageous combinations (groups of target genes) of modified target genes.

**Table 3**

| **Panel Number** | **Group of Target Genes** | **Panel Number** | **Group of Target Genes** |
|---|---|---|---|
| 1 | HMGB1, STUB1 | 79 | BACH2, ENTPD7, FCER1G, HLX, HMGB1, GATA1, IFI16, VEGFC |
| 2 | CAPN1, FOXO3, HMGB1, IFI16, KLHL22, LRRK2, PSAP, STUB1, DCP1B | 80 | CAPN1, CASP9, CDH1, HMGB1, PSMD2, STUB1 |
| 3 | DDIT4, HMGB1 | 81 | CD24, HMGB1, TSPAN32 |
| 4 | ARID1B, BRCC3, CCNG2, KLHL22, LATS2, NPM2, PML, SMARCD1, HMGB1, SWI5 | 82 | CD24, HMGB1, KLHL22, MAPKBP1, PML |
| 5 | ARID1B, GATA1, PML, PSMD2, SMARCD1 | 83 | CRLF2, FCER1G, HMGB1, IFI16, LRRK2, MAPK11, GATA1 |
| 6 | ARID1B, BRCC3, KLHL22, NPM2, PML, SMARCD1, LATS2 | 84 | CRLF2, HMGB1 |
| 7 | HORMAD2, NPM2 | 85 | HMGB1, IFI16, PML, XPR1 |
| 8 | NPM2, PKP4 | 86 | ENTPD7, HLX, HMGB1, VEGFC, ANGPT2, MIA3 |
| 9 | PKP4, VEGFC | 87 | PML, TRIM2 |
| 10 | BRCC3, PML, KLHL22, HMGB1, NDRG1 | 88 | ARID1B, CHD7, ENTPD7, FCER1G, FOXO3, GATA1, HLX, HMGB1, IFI16, SMARCD1, ZEB1 |
| 11 | DDIT4, KLHL22, LRRK2, NPM2 | 89 | HLX, HMGB1, CD24, BLOC1S6 |
| 12 | ANGPT2, BCAS3, HMGB1, MIA3, SPARC, VEGFC, MACF1 | 90 | ENTPD7, FCER1G, HLX, HMGB1, CHD7, ZEB1, BACH2, IFI16 |
| 13 | HMGB1, VEGFC, SPARC, ANGPT2, HLX, MIA3 | 91 | IFI16, PML, HMGB1, DDIT4 |
| 14 | ANGPT2, RALGAPA1, VEGFC, SMAD1 | 92 | CASP9, DDIT4, IFI16, PML, TSPAN32 |
| 15 | ARHGAP5, KIDINS220, PKP4, RALGAPA1 | 93 | CD24, HMGB1, LRRK2, MAPKBP1, STK25, TRAF7, EIF3E |
| 16 | CDH1, MACF1, PKP4, RILPL1, RIMS2, STK25 | 94 | ANGPT2, CASP9, COL4A2, DDIT4, FOXO3, PCK2, RXRA, VEGFC |
| 17 | LRRK2, MACF1, NDRG1, BCAS3 | 95 | CASP9, FOXO3, PSMD2, TRAF7 |
| 18 | CDH1, MACF1, PKP4, RIMS2, RILPL1, STK25, SLC12A2 | 96 | HMGB1, TF, TRAF7 |
| 19 | ADAMTS15, MAPK11, PTBP1, SMAD1, ZEB1 | 97 | ANGPT2, CDH1, MAPK11, VEGFC |
| 20 | ARHGAP5, GPC3, ZEB1 | 98 | MAPK11, STK25 |
| 21 | CD24, ZEB1 | 99 | GRIA2, LRTOMT |
| 22 | CDH1, CHD7, HLX, LATS2, MACF1, MAPK11, RIMS2, KLHL22 | 100 | CAPN1, RPL5, RPL7, RPS11, RPS19, RPL23A, RPL35 |
| 23 | CDH1, EXT1, JADE3, KLHL22, MACF1, PML, RIMS2 | 101 | C1orf131, CHD7, EIF3E, RPL23A, RPL35, RPL5, RPL7, RPS11, RPS19, TRAF7, SH3BGRL, MAPK11, PSMD2 |
| 24 | CDH1, GPC3, MAPK11, PML | 102 | CHD7, RPL23A, RPL35, RPL5, RPL7, RPS11, RPS19, EIF3E, FAH, MPST, PSMD2, SLC36A4, MAPK11 |
| 25 | CHD7, HLX, MAPK11, SMAD1, TENM4, EXT1, LATS2, PSAP | 103 | CHD7, RPL23A, RPL35, RPL5, RPL7, RPS11, RPS19, EIF3E, MAPK11, PSMD2, TRAF7 |
| 26 | COL4A2, GPC3, MIA3, TF | 104 | ADAMTS15, CAPN1, CASP9, BRCC3 |
| 27 | EBF1, MED11, RXRA, SMAD1 | 105 | BRCC3, PSMD2, SMAD1, STUB1 |
| 28 | EXT1, TCOF1 | 106 | CAPN1, LRRK2, STUB1 |
| 29 | FGF12, VEGFC | 107 | BCL2L12, CASP9, HMGB1, IFI16, SERPINI2 |
| 30 | FOXO3, LRRK2, NF2, VEGFC | 108 | CCNG2, GTF2H1, LATS2 |
| 31 | FOXO3, LRRK2, PTBP1, ZEB1 | 109 | CD24, CEMIP, LRRK2 |
| 32 | GATA1, PSMD2 | 110 | GATA1, NF2 |
| 33 | GPC3, HLX, HMGB1, PTPRM, SPARC, VEGFC, ZEB1 | 111 | MBD1, PML, RXRA |
| 34 | GRAP, VEGFC | 112 | PML, RPL5, SMAD1, STUB1 |
| 35 | MBD1, NDRG1, NF2, TENM4, VEGFC | 113 | PANK4, PTPRM, SSH3, PTBP1 |
| 36 | ANGPT2, SMAD1, VEGFC, MAPK11, MIA3 | 114 | HMGB1, IFI16, PML, BCL2L12, XPR1 |
| 37 | PTBP1, SMAD1 | 115 | CEMIP, LRRK2, CD24, ZDHHC20 |
| 38 | PTPRM, SPARC | 116 | ANGPT2, TF |
| 39 | FOXO3, GATA1, KDM5A, RPS19, RXRA, SRD5A1 | 117 | AREL1, FBXO30, MED11, PML, STUB1, TRAF7, TRIM2, UBE2Q1 |
| 40 | HOXB3, RXRA | 118 | CAPN1, FOXO3, MAPK11, KIDINS220 |
| 41 | EXT1, MAPK11, RXRB, SMAD1, VEGFC | 119 | FAM83A, PAG1 |
| 42 | CASP9, CYP2C8, MAPK11, RXRA, RXRB | 120 | FAM98B, SETBP1 |
| 43 | CASP9, FOXO3, GATA1, GPC3, IFI16, PML, RPS19, TF | 121 | GRAP, LZTR1 |
| 44 | CDH1, MAPK11, NF2 | 122 | LATS2, RPL5 |
| 45 | COL4A2, MAPK11, VEGFC, SMAD1 | 123 | PNPLA4, ZDHHC20 |
| 46 | FOXO3, LRRK2, NCOA7, STK25 | 124 | TLE1, TRAF7 |
| 47 | AKAP3, CHD7, GATA1, KIDINS220, LATS2, NPM2 | 125 | BRCC3, CASP9, FOXO3, NDRG1, PML |
| 48 | ANGPT2, CD24, EXT1, GPC3, SMAD1 | 126 | BLOC1S6, CADPS, LRRK2, RIMS2, TPRG1L, TMEM79, CHD7, PCK2, UBE2Q1 |
| 49 | CHD7, EXT1, GPC3, HOXB3, LRRK2, SLC25A25 | 127 | CASP9, CCNG2 |
| 50 | CHD7, FOXO3, GATA1, PSAP, SRD5A1, UBE2Q1 | 128 | DDIT4, FOXO3, IFI16, NDRG1, PML, RPL5 |
| 51 | EXT1, LATS2, TMEM79 | 129 | DDIT4, GTF2H1, MAPK11, NDRG1, PML |
| 52 | CHD7, EXT1, HOXB3, SLC25A25, LRRK2, DISP1 | 130 | DDIT4, IFI16, PML, TSPAN32, EXT1, MAPK11, XPR1, NDRG1 |
| 53 | ARID1B, FOXO3, HLX, HMGB1, MACF1, SMARCD1, STK25, TENM4, VEGFC | 131 | AKAP3, EXT1, GPC3, LATS2, PML, SMAD1, STUB1, ZEB1, COL4A2 |
| 54 | CAPN1, CASP9, GRIA2, LRRK2, MAPK11, PSMD2 | 132 | GPC3, LATS2, STUB1, VEGFC, ZEB1 |
| 55 | CASP9, ZEB1 | 133 | PML, RPL5, SMAD1, STUB1, PSMD2 |
| 56 | ADAMTS15, EXT1, GPC3, TCN2 | 134 | SMAD1, TF |
| 57 | ANGPT2, FOXO3, NPM2, PCK2 | 135 | ARID1B, HMGB1, HNF1A, MED11, SMARCD1, SET |
| 58 | CDS1, OSBPL8 | 136 | BACH2, ESX1, FOXO3, MXD3, TBX15, ZEB1 |
| 59 | CEMIP, EXT1, GLB1L2, GPC3, PCK2 | 137 | BACH2, GTF2H1, MED11, MXD3, RXRA, RXRB, SMAD1, TLE1 |
| 60 | CRTC3, SLC25A25 | 138 | BCAS3, CRTC3, GATA1, GTF2H1, HMGB1, NCOA7, RXRA, TLE1 |
| 61 | ALDH8A1, CYP2C8, MID1IP1, PCK2, RXRA | 139 | CCNG2, FOXO3, MAPK11, PCK2 |
| 62 | CYP2C8, PCK2, PNPLA4 | 140 | FOXO3, GATA1, HNF1A, HOXB3, RXRB, SMAD1 |
| 63 | DDIT4, FOXO3, LRRK2, SLC24A1 | 141 | FOXO3, TBX15 |
| 64 | ANGPT2, FOXO3, PCK2, EXT1, HNF1A | 142 | GTF2H1, TCOF1 |
| 65 | EXT1, GATA1, OSTC | 143 | BLOC1S6, CADPS, CHD7, HNF1A, LRRK2, PCK2, RIMS2, TPRG1L, UBE2Q1 |
| 66 | FAH, LRTOMT | 144 | BLOC1S6, CADPS, GATA1, LRRK2, MIA3, RIMS2, SYTL1, TMEM79, TPRG1L |
| 67 | FOXO3, LRRK2, ME2, MPST, NADK2, PCK2 | 145 | CDH1, LRRK2, PML |
| 68 | GLB1L2, PSAP | 146 | COL4A2, SLC36A2, SLC36A4 |
| 69 | GPC3, NADK2, PANK4, TCN2 | 147 | FGF12, FXYD7, SCN1A, SLC12A2, SLC24A1 |
| 70 | LRRK2, SLC25A25 | 148 | PSAP, SLC12A2, SLC25A25, SLC36A2, SLC36A4, SLC7A14, SLC24A1 |
| 71 | ME2, NADK2 | 149 | CDH1, LRRK2, PML, BACH2, LATS2, OSBPL8 |
| 72 | DDIT4, ME2, PCK2, GGCX | 150 | HMGB1, PSMD2, SPARC, TF, VEGFC |
| 73 | MED11, RXRA, RXRB, PCK2 | 151 | ANGPT2, CASP9, MAPK11, FCER1G, PML |
| 74 | PML, STK25 | 152 | HMGB1, IFI16, PTBP1, XPR1, ZDHHC20 |
| 75 | RXRA, SRD5A1 | 153 | CD24, EXT1, FOXO3, GPC3, GRK6, LATS2, LRRK2, MACF1, TLE1 |
| 76 | CYP2C8, PNPLA4, PCK2, ALDH8A1 | 154 | GPC3, LRRK2, MACF1, STUB1, NDRG1 |
| 77 | CRTC3, MAPK11, RXRA | 155 | PSMD2, TLE1 |
| 78 | ARID1B, BRD9, SMARCD1, GTF2H1, MAPK11, PML | | |

The gene sets as shown in **Table 3** were constructed from the enriched gene hit list by systematically integrating functional and pathway annotations from KEGG, GO, and Reactome databases. First, all retrieved annotations were assigned to each gene to capture molecular and cellular functions as well as pathway-level interactions. Genes were then grouped into sets based on shared functional annotations, ensuring that each set contained genes with similar roles in biological processes. To refine the gene sets, smaller groups fully contained within larger ones were removed while retaining their functional annotations. The remaining sets were further merged using the Jaccard similarity index (>50%) to reduce redundancy while preserving meaningful relationships. To ensure biological relevance, growth-related categories were specifically filtered, and only pathways related to essential cellular functions were retained. The terminology used to filter relevant sets is shown in **Table 4.**

**Table 4**

| **Category** | **Key Terms** |
|---|---|
| **Growth & Development** | growth, differentiation, division, proliferation, embryonic development, stem cell differentiation, tissue regeneration |
| **Cell Cycle & Regulation** | Cell Cycle, Cyclins, CDKs, p53, DNA Damage Response, CHK1, CHK2, apoptosis, survival, cycle |
| **Signaling Pathways** | TGF-beta, Transforming-Growth-Factor, MAPK, PI3K-Akt, ERK, mTOR, Ras, Wnt, JAK-STAT, NF-κB, Hedgehog, Notch |
| **Metabolism & Energy** | nutrients, metabolism, energy, Glycolysis, Pentose Phosphate Pathway, Oxidative Phosphorylation, AMPK, Lipid Metabolism |
| **Molecular Regulation** | Transcriptional Regulation, Chromatin Remodeling, Cytoskeletal Organization, NFAT, SMAD, HIF-1, FOXO |
| **Apoptosis & Stress Response** | Bcl-2, TNF, Fas, caspase, Bax, Bak, p21, p27, oxidative stress, ROS, death receptor, autophagy |
| **Immune & Cytokine Signaling** | cytokine, IL-, interferon, IFN-, permeability, mitochondria, ER stress, cytochrome c, perforin, granzyme, immune response, inflammation |

As a result, the gene sets consist of genes that either share molecular or cellular functions or operate within the same biological pathways relevant to growth. These sets are likely to show synergistic interactions for efficient functional engineering of faster growing CHO cells.

One embodiment relates to the modified CHO cell, wherein the at least one modification of at least one endogenous coding sequence is combined with at least one modification of at least one different endogenous coding sequence of a target gene selected from the group of HMGB1, STUB1, CAPN1, FOXO3, IFI16, KLHL22, LRRK2, PSAP, DCP1B, DDIT4, ARID1B, BRCC3, CCNG2, LATS2, NPM2, PML, SMARCD1, SWI5, GATA1, PSMD2, HORMAD2, PKP4, VEGFC, NDRG1, ANGPT2, BCAS3, MIA3, SPARC, MACF1, HLX, RALGAPA1, ARHGAP5, KIDINS220, CDH1, RILPL1, RIMS2, STK25, SLC12A2, ADAMTS15, MAPK11, PTBP1, SMAD1, ZEB1, CD24, CHD7, EXT1, JADE3, TF, EBF1, MED11, RXRA, TCOF1, FGF12, NF2, NCOA7, AKAP3, ME2, MPST, NADK2, PCK2, GPC3, PTPRM, GRAP, MBD1, RXRB, CASP9, CYP2C8, RPS19, HOXB3, NDRG1, FAH, LRTOMT, CDS1, OSBPL8, CEMIP, GLB1L2, CRTC3, SLC25A25, ALDH8A1, MID1IP1, HNF1A, OSTC, PANK4, GGCX, GTF2H1, SET, BACH2, ENTPD7, FCER1G, ESX1, MXD3, TBX15, GRIA2, AREL1, FBXO30, TRIM2, TLE1, TRIM2, UBE2Q1, RPL5, RPL7, RPS11, RPL23A, RPL35, SH3BGRL, SLC36A4, XPR1, ZDHHC20, MAPKBP1, TRAF7, EIF3E, BLOC1S6, CADPS, SYTL1, TPRG1L, SSH3, DISP1, GRK6, LZTR1, PAG1, SCN1A, FXYD7, TSPAN32, MAPKBP1, MAPKBP1, MAPKBP1, or of any homologue, orthologue, or paralogue thereof, preferably of a group of target genes from an individual panel as defined in **Table 3;** and the at least one combination of modifications leads to the reduced transcription and/or reduced functional expression of said at least one endogenous protein, or of any homologue, orthologue, or paralogue thereof.

One embodiment relates to the modified CHO cell, wherein said at least one endogenous protein has an amino acid sequence corresponding to SEQ ID NO: 1 to 235, or any homologue, orthologue, or paralogue thereof having an amino acid sequence having at least 75%, 76%, 77%, 78%, 79%, 80%, 81%, 82%; 83%, 84%, 85%, 86%, 87%, 88%, 89%, 90%, 91%, 92%, 93%, 94%, 95%, 96%, 97%, 98% or 99% sequence identity to a sequence corresponding to SEQ ID NO: 1 to 235, respectively.

Endogenous proteins represented by their respective SEQ ID NO: 1 to 235 are listed in **Table 1.**

One embodiment relates to the modified CHO cell, wherein the total concentration of the at least one modified endogenous protein is individually reduced by at least 20%, 25%, 30%, 35%, 40%, 45%, 50%, more preferably at least 55%, 60%, 65%, 70%, 75%, 80%, most preferably at least 80%, 81%, 82%, 83%, 84%, 85%, 86%, 87%, 88%, 89%, 90%, 91%, 92%, 93%, 94%, 95%, 96%, 97%, 98%, 99%, 100%, or even 100%, as determined by measuring the relative concentration of the at least one modified endogenous protein in ng/mL per sample obtained in a modified CHO cell in comparison to a reference sample obtained from a CHO wild-type cell not carrying the at least one modification in its genome.

One embodiment relates to the modified CHO cell, wherein said endogenous protein has a reduced transcription and/or reduced functional expression, wherein the modified CHO cell comprises at least one modification in at least three, at least four, at least five, at least six, at least seven, at least eight, at least nine, at least ten, at least eleven, at least twelve, at least thirteen, at least fourteen, at least fifteen, at least sixteen, at least seventeen, at least eighteen, at least nineteen, at least twenty or more different endogenous coding sequences encoding at least one endogenous protein and at least one recombinant sequence encoding at least one recombinant protein and/or at least one recombinant RNA.

One embodiment relates to the modified CHO cell, wherein said CHO cell comprises at least one recombinant gene encoding at least one recombinant protein and/or encoding at least one recombinant RNA molecule.

One embodiment relates to the modified CHO cell, wherein said at least one recombinant protein is a therapeutic molecule.

One embodiment relates to the modified CHO cell, wherein said at least one modification is selected from the group consisting of at least one insertion, at least one deletions, and at least one substitution, including a base edit, or any combination thereof, preferably wherein said at least one modification is present in exon 1 of the respective endogenous coding sequence, and/or wherein said at least one modification in said endogenous sequence is a frame-shift mutation or a point mutation, the point mutation resulting in a stop codon.

In one embodiment of the present invention, the at least one modification may lead to premature transcription termination resulting in a truncated and thus inactive form of the respective protein. Preferably, the at least one modification may lead to premature transcription termination in exon 1 of the respective protein. In another preferred embodiment, the mutation may be in another exon, e.g., in case another exon is easier accessible for successful and efficient editing.

Preferably, premature transcription termination may occur in the first 50%, preferably in the first 40%, preferably in the first 30%, preferably in the first 20%, preferably in the first 10%, preferably in the first 5%, preferably in the first 2% of the respective genomic coding sequence viewed from its transcriptional start, i.e. its start codon.

By prematurely terminating the transcription of a given target gene as close to its respective transcriptional start as possible the amount of formed transcript is kept to a minimum and the overall load of the respective protein in the modified CHO cell according to the present invention is minimized.

One embodiment relates to the modified CHO cell, wherein said at least one modification in said at least one endogenous coding sequence allows for an optimized production of the at least one recombinant protein and/or at least one recombinant RNA, (i) wherein said optimized production of the at least one recombinant protein and/or the at least one recombinant RNA is characterized by an increase (a) in the yield of the at least one recombinant protein, and/or (b)in the yield of the at least one RNA encoding said at least one recombinant protein cell, and/or (c) in the yield of the at least one recombinant RNA, in comparison to a reference sample obtained from a CHO wild-type cell not carrying the at least one modification in its genome, wherein the total concentration of the at least one modified endogenous protein is reduced by at least 20%, 25%, 30%, 35%, 40%, 45%, 50%, more preferably at least 55%, 60%, 65%, 70%, 75%, 80%, most preferably at least 80%, 81%, 82%, 83%, 84%, 85%, 86%, 87%, 88%, 89%, 90%, 91%, 92%, 93%, 94%, 95%, 96%, 97%, 98%, 99%, 100%, or even 100%, as determined by measuring the relative concentration of the at least one recombinant protein and/or at least one recombinant RNA in ng/mL per sample obtained in a modified CHO cell in comparison to a reference sample obtained from a CHO wild-type cell not carrying the at least one modification in its genome.

One embodiment relates to the modified CHO cell according to the first aspect or any embodiment thereof, wherein all alleles of said at least one endogenous coding sequence comprise at least one or more of said at least one modification.

One embodiment relates to the modified CHO cell according to the first aspect or any embodiment thereof, wherein The modified CHO cell comprises at least one modification in at least three, at least four, at least five, at least six, at least seven, at least eight, at least nine, at least ten, at least eleven, at least twelve, at least thirteen, at least fourteen, at least fifteen, at least sixteen, at least seventeen, at least eighteen, at least nineteen, at least twenty or more different endogenous coding sequences encoding at least one endogenous protein and at least one recombinant sequence encoding at least one recombinant protein and/or at least one recombinant RNA, wherein: each endogenous coding sequence is selected from the group consisting of the following KEGG pathways: ribosome, spliceosome, cell cycle, nucleocytoplasmic transport, oxidative phosphorylation, biosynthesis of cofactors, mRNA surveillance pathway, nucleotide excision repair, RNA degradation, proteasome, DNA replication, basal transcription factors, RNA polymerase, homologous recombination and mismatch repair.

KEGG pathways provide a structured framework for classifying genes based on their roles in fundamental cellular processes. Several KEGG pathways are crucial for maintaining cellular integrity, gene expression, protein synthesis, and genome stability. The ribosome pathway consists of genes encoding ribosomal proteins and assembly factors essential for protein synthesis. Closely related is the spliceosome pathway, which involves genes responsible for pre-mRNA splicing, ensuring proper maturation of mRNA before translation. Complementary to these is the mRNA surveillance pathway, which includes genes that detect and degrade defective mRNA, preventing the production of faulty proteins. In gene expression, the RNA polymerase pathway involves genes encoding RNA polymerase subunits that catalyze transcription, while the basal transcription factors pathway encompasses genes that regulate RNA polymerase activity to initiate transcription. The nucleocytoplasmic transport pathway plays a role in moving mRNA, proteins, and other molecules between the nucleus and cytoplasm, ensuring proper gene expression and cellular function. Genomic stability is maintained through pathways that regulate DNA replication and repair. The DNA replication pathway includes genes essential for replication origin recognition, DNA polymerase activity, and replication fork progression. In response to DNA damage, the homologous recombination pathway and the mismatch repair pathway function to correct double-strand breaks and replication errors, respectively. The nucleotide excision repair pathway is specifically involved in repairing bulky DNA lesions, such as those caused by UV radiation. Energy production and metabolic regulation are governed by the oxidative phosphorylation pathway, which comprises genes encoding components of the electron transport chain responsible for ATP production in mitochondria. Additionally, the biosynthesis of cofactors pathway involves genes required for the synthesis of essential cofactors, such as vitamins and enzyme cofactors that support numerous biochemical reactions. Protein homeostasis and degradation are controlled by the proteasome pathway, which consists of genes encoding proteasomal subunits responsible for degrading misfolded or damaged proteins to maintain cellular function. Similarly, the RNA degradation pathway ensures the removal of defective or unnecessary RNA molecules, preventing the accumulation of non-functional transcripts. The cell cycle pathway integrates genes that regulate cell division, ensuring proper progression through different phases and preventing uncontrolled proliferation. This pathway interacts closely with DNA replication and repair mechanisms to maintain genomic integrity during cell division.

Advantageously, these KEGG pathways classify genes into essential biological processes, providing insights into cellular regulation, gene function, and disease mechanisms. Their integration into functional networks allows for a systematic understanding of how genes contribute to cellular homeostasis and overall organismal health.

One embodiment relates to the modified CHO cell according to the first aspect or any embodiment thereof, wherein the CHO cell undergoes a measurable and quantifiable enhancement of cell size (volume or mass) beyond that observed in a suitable reference or control population, preferably derived by using peak fold-change values to predict growth rate improvements.

In certain embodiments the modified CHO cell may undergo a single gene predicted growth delta of at least 2%, at least 3%, at least 4%, at least 5%, or at least 6%.

In certain embodiments the modified CHO cell may undergo a gene set combined KO growth delta of at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, or at least 100%.

The single gene growth delta refers to the predicted increase in cell growth rate resulting from the knockout of an individual gene in Chinese Hamster Ovary (CHO) cells. This is determined by analyzing peak fold-change values in growth following the deletion of each gene. Each gene is evaluated independently, and the resulting growth delta reflects the isolated effect of that specific gene knockout on cellular proliferation.

In contrast, the gene set combined growth delta represents the cumulative predicted growth improvement obtained by combining several individual gene knockouts.

Chinese hamster ovary (CHO) cells are widely recognized as a principal host system for the industrial production of recombinant proteins, antibodies, and other biologically active molecule. Advantageously, even moderate enhancements in growth, when properly matched with stable protein expression, can lead to substantial gains in manufacturing capacity, cost efficiency, and ultimate product throughput.

Example 3 relates to the methodology underlying cell growth evaluation, with experimental results illustrated in FIG 11.

A second aspect relates to a method for the production of at least one modified CHO cell, comprising the steps: (i) providing at least one CHO cell comprising at least one endogenous target nucleic acid segment; (ii) providing at least one genome or transcriptome editing agent comprising at least one RNAi agent, or at least one site-directed endonuclease, preferably being selected from a meganuclease, a ZFN, a TALEN, a CRISPR-nuclease, or a nickase or nuclease-dead variant therefrom, or a nucleic acid molecule encoding the same, and optionally in case of a CRISPR-nuclease: providing at least one suitable, functional guide RNA molecule, or a nucleic acid molecule encoding the same; (iii) introducing into said at least one CHO cell the at least one genome editing agent of step (ii); (iv) obtaining at least one modified CHO cell comprising at least one modification in said at least one endogenous target nucleic acid segment according to step (i); (v) optionally: selecting a further target nucleic acid segment and repeating steps (i) to (iv) at least one time to obtain at least one modified CHO cell comprising at least one further modification in a further endogenous target nucleic acid segment.

In a preferred embodiment of the method according to the present invention, the at least one endogenous target nucleic acid segment may be a nucleic acid sequence, preferably a DNA sequence, coding for part of any of the amino acid sequences according to any of the SEQ ID NOs: 1 to 235.

In a preferred embodiment of the method according to the present invention, a CRISPR/Cas system may be used as genome editing system. This clustered regularly interspaced short palindromic repeats (CRISPR) and CRISPR-associated protein (CRISPR/Cas) system has significantly altered molecular biology research and development over the last decade offering enormous possibilities to modify cells of interest, including complex eukaryotic cells, in a highly targeted way. Any CRISPR/Cas system suitable for eukaryotic cell genome editing can be used according to the present disclosure. The CRISPR/Cas system may include a CRISPR/Cas9 systems, a CRISPR/Cas13 systems, CRISPR/Cas12a system (also called CRISPR/Cas12a system), a CRISPR/C2C2 systems, CRISPR/CasX systems (also called CRISPR/Cas12e system), CRISPR/CasY systems, CRISPR/Cmr systems, CRISPR/Csm systems, CRISPR/MAD2 systems, CRISPR/MAD7 systems, CRISPR/CasZ systems, CRISPR/Cas14a, b or c systems, CRISPR/Cas phi systems, CRISPR/Cas12f systems, or catalytically active fragments or variants thereof. Various kinds of CRISPR systems and optimized mutated systems, including nickase or nuclease-dead based systems, CRISPR mutants with optimized PAM-specificities and temperature tolerance, and various methods of using these CRISPR variants and mutants alone or on combination with other effectors, e.g., to be suitable for base editing and prime editing, have been developed and are suitable to be used according the present disclosure (cf. Jinek, et al., A Programmable Dual-RNA-Guided DNA Endonuclease in Adaptive Bacterial Immunity. Science 2012, 337, 816-821; Hillary and Ceasar, Mol Biotechnol. 2022 Sep 27;1-15, doi: 10.1007/s12033-022-00567-0; Zetsche, et al. Cpf1 Is a Single RNA-Guided Endonuclease of a Class 2 CRISPR-Cas System. Cell 2015, 163, 759-771; Kim et al. Nat Biotechnol. (2022);40(1):94-102; doi: 10.1038/s41587-021-01009-z; Liu et al., CRISPR J. April 2020; 3(2): 97-108.; Karvalis et al. Nucleic Acids Res. (2020); 48(9):5016-5023. doi: 10.1093/nar/gkaa208, Selkova et al. RNA Biol. (2020); 17(10):1472-1479; doi: 10.1080/15476286.2020.1777378, Rees and Liu, 2018. Nat. Rev. Genet.; doi: 10.1038/s41576-018-0059-1, Komor et al., Nature 533, 420-460, 2016; Komor et al., 2017, Science Advances, doi:10.1126/sciadv.aao4774M; Gaudelli et al., Nature, 551: 464-471, 2017; Lu et al., Int. J. Mol. Sci. 2022, 23, 9862. https://doi.org/10.3390/ijms23179862).

As it is known to one skilled in the art, CRISPR/Cas effectors need a guiding molecule to exert their activity. The "guide molecule", in particular the "guide RNA" (gRNA) may comprise a trans-activating CRISPR RNA (tracrRNA) and a CRISPR RNA (crRNA). Certain CRISPR nucleases, e.g., Cas12a, only require a crRNA. TracrRNA and crRNA can be combined in one molecule, resulting in a single guide RNA (sgRNA), which comprises the sequence information targeting the genomic sequence for cleavage by the nuclease. For multiplexing purposes, more than one gRNA allowing the recognition and thus modification of more than one target site in a genome may be designed and used in one and the same experiment, or one after another.

In a preferred embodiment of the method according to the present invention, the CRISPRnuclease may be a Class 2 Type V nuclease, or a variant or mutant thereof, including a Cas12a (formerly known as Cpf1) or a MAD7 or a Cas14 effector. Suitable vectors and plasmids to express the relevant CRISPR effector and the cognate gRNA(s) are available to the skilled person and can be easily adapted by designing the gRNA(s) *in silico.*

In another embodiment, an RNAi agent can be used to knock-down or silence at least one nucleic acid coding for at least one target gene of interest. For RNAi, small RNAs function to guide specific effector proteins to a target nucleotide sequence by complementary base pairing resulting in degradation of the target. A "gene silencing construct" or "RNAi agent" usually comprises so called "sense" and "antisense" sequences. Sense and antisense sequences are complementary sequences, which are present in reverse orientation in a nucleic acid sequence. If a nucleic acid construct comprises a sense and a corresponding antisense sequence, the two complementary sequences form an RNA double strand upon transcription, which results in an "RNA hairpin". With these RNA hairpin sequences, more than one target gene can be knocked-down simultaneously in certain embodiments.

In certain embodiments, a knock-down of at least one target gene via, for example, an RNAi agent and a knock-out mediated by genome editing, including CRISPR/Cas, may be combined to reduce the expression of the at least one target gene.

Certain embodiments relate to a method for the production of at least one modified CHO cell comprising the step: introducing into said at least one CHO cell the at least one genome editing agent via a suitable delivery method, such as instance transfection, lipofection, viral transduction, preferably electroporation, but not limited thereof.

Certain embodiments relate to a method for the production of at least one modified CHO cell by a genome-wide CRISPR knockout screen, wherein a lentiviral library targeting the full expressed genome of CHO cells is introduced into CHO cell pools stably expressing a Cas9 nuclease. The method may include: (i) performing viral transduction at a low multiplicity of infection (MOI) to favour single-guide integration, followed by an antibiotic selection step, preferably puromycin to enrich for transduced cells; (ii) cultivating the enriched population for multiple weeks with periodic sampling, preferably at least every 14 days; (iii) extracting and sequencing genomic DNA and of integrated sgRNAs at each time point, preferably by next-generation sequencing (NGS); and (iv) identifying candidate genes beneficial or detrimental to growth by comparing sgRNA enrichment or depletion over time.

Advantageously, viral transduction may be performed at an exceptionally low multiplicity of infection (MOI), while a greater number of virus particles than typically used is applied. This strategy minimized the occurrence of multiple-copy insertions while maintaining high efficiency in sgRNA delivery, ensuring a more accurate and comprehensive representation of genetic perturbations. Furthermore, rather than relying on incomplete or fragmented reference genomes, the actual sequenced CHO genome may be utilized, allowing for precise targeting across the entire expressed genome.

Stable Cas (e.g., Cas9) expression may be employed instead of transient expression, ensuring consistent editing efficiency and ease the transfection of billions of cells. This approach enables high library coverage, ensuring that all expressed genes were thoroughly interrogated for their potential role in increasing CHO cell proliferation. Copy number validation may be rigorously performed using orthogonal methods, evaluating hundreds of clones to confirm single-copy integrations with high confidence.

Advantageously, significantly greater sequencing depth per sgRNA/gene may is achieved in comparison to prior methods, leading to improved accuracy, increased sensitivity in detecting true genetic effects, and greater confidence in the results.

By implementing low-MOI transduction, stable Cas expression, comprehensive genome targeting according to the present invention, and meticulous validation strategies, the present invention establishes a new standard in CHO genome engineering. The ability to develop CHO cells with enhanced growth rates directly benefits the biopharmaceutical industry by reducing production timelines and increasing recombinant protein yields. Through greater accuracy, reproducibility, and efficiency, this approach accelerates CHO cell line development, improving the scalability and cost-effectiveness of biologic manufacturing.

Certain embodiments relate to a method for the production of at least one modified CHO cell by engineering a CHO cell with an improved growth phenotype by knocking out one at least one identified target gene, comprising the following steps: (i) the at least one identified target gene is independently screened by designing a precise CRISPR single-guide RNA set (sgRNA-set) directed to the gene of interest; (ii) the CHO cells are transfected or transduced with the sgRNA, followed by expansion under suitable selection; and (iii) the improved growth phenotype is verified in a shake-flask culture or a perfusion bioreactor.

Advantageously, knockout target genes does not impair upstream processing (USP) performance or product yield in a manufacturing context. In these embodiments, the gene-edited CHO cells are subjected to additional bioprocess-relevant tests. Cells producing a desired recombinant molecule may be cultivated under conditions typical of an industrial process (e.g., fed-batch or perfusion). Product titers, post-translational modifications (e.g., glycosylation profiles), and viability may be compared between the gene-edited cell line and an unmodified control line.

In certain embodiments, sequential or multiplexed knockouts, wherein multiple target gene knockouts are combined. A target gene of interest may be disrupted, and a cell clone with the desired phenotype may be isolated. Additional target genes, or a panel of target genes, may be additionally knocked out using CRISPR reagents. Then, the resulting cell lines, each carrying multiple independent knockouts, may be assessed for cumulative benefits to cell growth, viability, and productivity.

Certain embodiments relate to a refined the screening and engineering process by integrating additional or refined filtering criteria to identify low-abundance or borderline candidates that may be overlooked in stringent analyses. Such an approach may uncover additional genes that positively or negatively influence CHO cell performance when knocked out.

One embodiment relates to the method according to the second aspect, wherein (i) in step (ii) of the second aspect a CRISPR-nuclease is used; and (ii) optionally: wherein a Cas enzyme is constitutively expressed by said at least one modified CHO cell; and (iii) wherein the at least one suitable functional guide RNA molecule comprises a sequence corresponding to any of the nucleic acid sequences according to SEQ ID NOs: 236 to 1636 or corresponding to a nucleic acid sequence having at least 75%, 76%, 77%, 78%, 79%, 80%, 81%, 82%; 83%, 84%, 85%, 86%, 87%, 88%, 89%, 90%, 91%, 92%, 93%, 94%, 95%, 96%, 97%, 98% or 99% sequence identity to any one of the sequences according to SEQ ID NOs: 236 to 1636.

In preferred embodiments the Cas enzyme is Cas9.

A functional guide RNA typically comprises two main components: a short region (usually around 20 nucleotides) complementary to the target DNA sequence and a structural region (often termed the scaffold or tracrRNA portion). When combined with a Cas endonuclease, the guide RNA guides the Cas enzyme (e.g., Cas9) to the intended DNA locus. The 20-nucleotide "guide" region within the sgRNA determines specificity. This region hybridizes with the DNA sequence of interest through standard Watson-Crick base pairing, thereby determining the exact genomic site for cleavage. For Cas9, efficient targeting also depends on a protospacer adjacent motif (PAM), typically a short DNA sequence immediately downstream of the target site (e.g., 5'-NGG-3' for the commonly used SpCas9). The presence and position of the PAM sequence relative to the guide region can significantly influence editing efficacy. Once the sgRNA:Cas complex engages the target site, the Cas enzyme catalyzes a double-stranded break (DSB) or, depending on the Cas variant, a nick in one strand of the DNA. Subsequent repair events via non-homologous end joining (NHEJ) or homology-directed repair (HDR) can introduce deletions, insertions, or more precise edits. Multiple sgRNAs can be designed to target several genomic sites simultaneously. This multiplexing capability streamlines complex genomic modifications, such as simultaneous perturbation of multiple genes (Cong L et al., Multiplex Genome Engineering Using CRISPR/Cas Systems., Science (2013), 339(6121):819-823).

Advantageously, by customizing the 20-nucleotide guide region, researchers can direct Cas enzyme to virtually any DNA sequence of interest, limited mainly by the presence of a suitable PAM. Furthermore guide RNA sequences are relatively short and easily synthesized, making them cost-effective and convenient for large-scale applications, such as high-throughput genetic screens. Guide RNA-based designs allow swapping out of the guide sequence without altering the core Cas enzyme. This modularity accelerates prototyping and iterative experiments.

A third aspect relates to a method for the production of at least one recombinant molecule, preferably at least one recombinant protein and/or recombinant RNA, comprising the steps: (a) providing at least one modified CHO cell according to the first aspect or any embodiment thereof, wherein the at least one modified CHO cell comprises at least one recombinant gene encoding at least one recombinant protein, DNA or RNA; (b) culturing said at least one target cell in a culture medium such that at least one recombinant molecule is transcribed and/or translated; (c) said at least one recombinant molecule; (d) purifying and/ or decontaminating said at least one recombinant molecule, preferably the at least one recombinant protein.

In a preferred embodiment of the method according to the present invention, the at least one recombinant gene is exogenously expressed from at least one expression vector, i.e. at least one plasmid. Said expression vector may be particularly selected from the group consisting of mammalian expression vectors, as these are disclosed herein, or as these are known to one skilled in the art. The skilled person can thus easily define suitable expression vectors for a host cell of interest in his/her respective technical field that are compatible with the present disclosure.

In a preferred embodiment of the method according to the present invention, culturing said at least one target cell may be performed by a method selected from the group consisting of batch cultivation, fed-batch cultivation, perfusion cultivation, and the like, and combinations thereof. In a preferred embodiment of the method according to the present invention, the culture medium for culturing said at least one target cell may be selected from the group consisting of, for example, a non-chemically defined or a chemically-defined cell culture medium, but not limited thereof.

In one embodiment of the method according to the present invention, purifying and/or decontaminating may comprise one or more process steps selected from the group consisting of affinity purification, ion exchange, hydrophobic interaction chromatography, and size exclusion chromatography, but not limited thereof.

In a preferred embodiment of the method according to the present invention, the at least one recombinant protein of interest may be selected from the group consisting of, but not restricted to, therapeutic proteins, monoclonal antibodies, bispecific antibodies, fusion proteins, peptibodies, and peptides.

A fourth aspect relates to the use of at least one modified CHO cell according to the first aspect or any embodiment thereof for the production of at least one pharmaceutical drug.

Certain embodiments relate to the use of at least one modified CHO cell according to the first aspect or any embodiment thereof for the production of at least one pharmaceutical drug, or a part thereof.

The pharmaceutical drug can be any biomolecule, preferably a recombinant molecule, including recombinant proteins, peptide, RNA, DNA, or any combination, fusion molecules, including covalently or non-covalently (e.g., hybridized; antigen::antibody complexes etc.) tethered molecules, that can be produced in a CHO cell of the present disclosure. The pharmaceutical drug will preferably be purified. Further, the pharmaceutical drug may be further modified, e.g., by chemical synthesis to attach an additional moiety, or to link two molecules to each other. Further, the pharmaceutical drug may be stabilized, lyophilized, and provided together with suitable buffers and agents all being pharmaceutically acceptable. Galenic techniques for compounding a pharmaceutical drug are known to one skilled in the art.

A fifth aspect relates to a kit comprising (a) at least one modified CHO cell, the modified CHO cell or the kit comprising: (b.i) at least one nucleic acid molecule suitable for expressing at least one recombinant molecule of interest, preferably at least one recombinant protein of interest; optionally means for introducing the same into the modified CHO cell genome, or alternatively (b.ii) alternatively at least one nucleic acid molecule encoding at least one recombinant molecule, preferably at least one recombinant protein; optionally means for introducing the same into the modified CHO cell genome; or (b.iii) alternatively: at least one nucleic acid molecule suitable for transcribing at least one recombinant RNA molecule; optionally means for introducing the same into the modified CHO cell genome; or (b.iv) alternatively: at least one nucleic acid molecule encoding at least one recombinant RNA molecule; optionally means for introducing the same into the modified CHO cell genome; and (c) optionally: culture medium suitable for the at least one modified CHO cell according to step (a); and (i) to replicate the at least one nucleic acid molecule according to any of the steps (b.i), (b.ii), (b.iii), and (b.iv) endogenously or exogenously; and (ii.a) to express the at least one recombinant according to step (b.i) and/or (b.ii), and/or to transcribe the at least one recombinant RNA molecule according to step (b.iii) and/or (b.iv).

In certain embodiment there is provided a recombinant molecule as pharmaceutical agent, preferably a recombinant protein or peptide, including at least one monoclonal antibody, obtained from a modified CHO cell according to the present invention for use in a method of treating a subject in need thereof, including preventing a disease and curing a disease.

Further provided are methods for using the purified recombinant molecules, alone or in combination with further pharmaceutically acceptable ingredients and buffers, as produced by the modified CHO cells of the present invention in a method of treating a subject in need thereof.

Further aspects and embodiments of the present invention can be derived from the subsequent, non-limiting, detailed description, the Figures, the Sequence Listing, as well as the attached set of claims.

### Examples

### Example 1: Design and workflow of a genome-wide lentiviral knockout screen

A comprehensive lentiviral whole-genome knockout (KO) screen was conducted to identify relevant KO targets. The experimental setup included four approaches, three of which were cultivated in shake flasks, while one was maintained in a perfusion bioreactor to achieve higher library coverage. In all approaches, cells were infected with the whole expressed genome covering CHO genes (17,759 genes, 109,543 sgRNAs). Each gene was targeted by six sgRNAs, and the library also contained non-targeting (2,021) and intron-targeting sgRNAs (1,834) as controls.

The experimental workflow, including viral transduction, selection, and analytics, is outlined in FIG 1. Stable Cas-expressing CHO DG44 cell pools were transduced with a lentiviral VSV-G virus-like particle library at a multiplicity of infection (MOI) of 0.15. Following transduction, puromycin selection was applied, and the selected population was monitored using a GFP-T2A-PuroR reporter via flow cytometry. Upon successful transduction, the gene targeted by the introduced sgRNA was knocked out, resulting in the corresponding KO phenotype. By analyzing the distribution of sgRNA read counts, it was possible to determine whether gene knockout had a beneficial or detrimental impact on cell growth. Genes whose knockout enhanced CHO cell proliferation exhibited an increase in their corresponding sgRNA read counts. This enrichment, or conversely, depletion of sgRNAs, was effectively monitored using next-generation sequencing (NGS) of the sgRNA inserts. Importantly, KO targets that conferred a growth advantage became enriched within the population, directly linking gene disruption to cell growth dynamics.

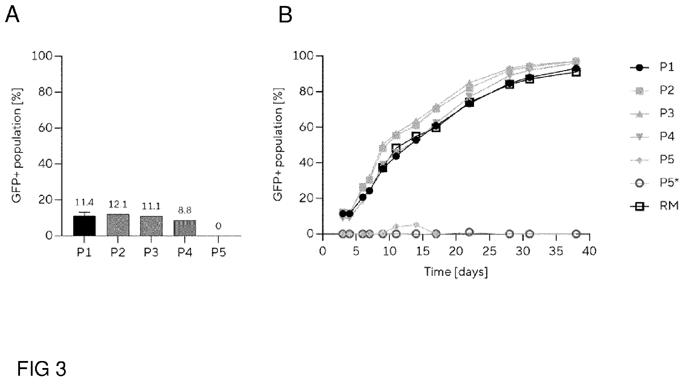

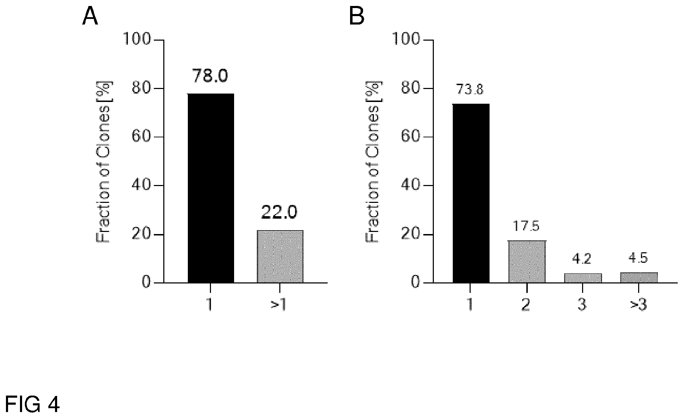

The library pool was cultivated for six weeks, with DNA extracted at two-week intervals. Illumina HiSeq-NGS sequencing was performed to quantify sgRNA read counts at each time point. After the majority of uninfected clones had been eliminated from the library, single-cell clones were generated to facilitate further analysis, including Sanger sequencing of sgRNA inserts and digital droplet PCR (ddPCR) for copy number variation (CNV) analysis. The cellular KO library was successfully established, as confirmed by the initial GFP-positive cell ratio and the enrichment of GFP-positive cells over time (FIG 3). Copy number analysis using Sanger sequencing and ddPCR produced similar results, with single-copy sgRNA integration observed in 78.0% and 73.8% of clones, respectively (FIG 4). This outcome represents a substantial improvement over conventional lentiviral whole-genome KO screens, which typically achieve only 40% single-copy integrations (Xiong et al., 2021).

A differential analysis was performed, as illustrated in Figure 2. Read count distributions were compared between the plasmid library and samples collected at two-, four-, and six-week time points. Additionally, the three late time points were treated as pseudo-replicates to enable a robust comparison with the plasmid library. Samples from shake-flask and perfusion cultivation modes were analyzed as independent replicates and compared against the plasmid library.

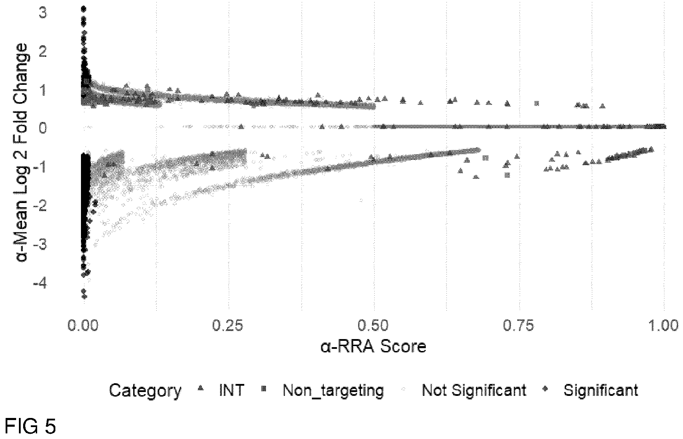

FIG 5 displays the differential gene abundance analysis, highlighting both positive and negative selection. Genes were categorized based on significance, along with non-targeting and intron-targeting groups. Cutoff criteria were established using all intron-targeting sgRNAs, as these should not significantly affect CHO cell growth and, therefore, serve as indicators of background noise in the screen. Random enrichment and depletion were expected to affect both gene-targeting and intron-targeting sgRNAs equally, making them a suitable benchmark. The cutoff criteria for significant hits are shown in **Table 4.**

**Table 4**

| **Type of Hit** | **Filter Criteria** |
|---|---|
| **Positive Selection (enriched)** | pos.fdr < 0.05 OR (pos.p.value < 0.025 AND pos.lfc > 1) |
| **Negative Selection (depleted)** | neg.fdr < 0.05 OR (neg.p.value < 0.025 AND neg.lfc < 0) |

In the time-point replicate comparison, 2,222 genes were identified as depleted, while 133 genes were enriched. The counts of enriched and depleted genes from all comparisons are summarized in **Table 5.**

**Table 5**

| **Comparison** | **Depleted Genes** | **Enriched Genes** |
|---|---|---|
| 2 Week vs. Plasmid | 1527 | 496 |
| 4 Week vs. Plasmid | 2323 | 122 |
| 6 Week vs. Plasmid | 2289 | 135 |
| Time Replicates | 2222 | 133 |
| Perfusion | 2367 | 299 |
| Shake Flask | 2314 | 110 |
| Unique Across Comparisons (No Filtering) | 3119 | 932 |
| **Unique Across Comparisons (Quality filter and ranking Filtering)** | **2467** | **219** |

KEGG pathway analysis of genes depleted across at least two comparisons revealed significant enrichment in pathways essential for cell survival, including those involved in the cell cycle, ribosome function, spliceosome activity, RNA degradation, and proteasome function (FIG 6). These findings underscore the crucial biological processes affected by gene knockouts and provide valuable insights into the genetic factors that drive CHO cell survival.

To generate a final hit list of genes whose KO resulted in increased cell growth, genes were ranked and scored based on multiple factors. Sequencing quality metrics, including sample purity before sequencing, median read counts, standard deviation, skewness, and low count percentage, were normalized across all samples and scored. A composite quality score was then calculated for each sample by applying weighted coefficients to these metrics (Purity = 0.05; Median = 0.15; Standard Deviation = 0.45; Skewness = 0.15; Low Count Percentage = 0.3).

The composite quality score was determined using the following equation: Quality Score = (wpurity × norm_purity) + (wmedian × norm_median) + (wsd × norm_sd) + (wskewness × norm_skewness) + (wlow count pct × norm_low count pct)

To standardize the dataset, sample quality scores were scaled from 0 to 1. Subsequently, the quality score for each comparison was calculated by incorporating the scores of all relevant samples. Additionally, the number of replicates was factored in to assign greater weight to hits that appeared consistently across multiple comparisons.

The relative score was computed using the equation: $Relative Score = Average Scaled Quality Score \times 0.1 \times Replicate Count$

Final scores were obtained by integrating the comparison-based relative score and multiplying the log fold change of each gene by its respective weight. These weighted log fold changes were then summed to establish the final prioritization of gene hits, as outlined in Equations (1) and (2). ${S}_{i} = {\sum }_{j} \left[{w}_{j}∗\frac{\left|{LFC}_{ij}\right|}{min \left\{\left|{LFC}_{ik}\right|:{LFC}_{ik}\neq 0,∀k\right\}}δ\left({LFC}_{ij}\neq 0\right)\right]$ (1) $δ \left({LFC}_{ij}\neq 0\right) = \left\{\begin{matrix}1 if {LFC}_{ij} \neq 0 \\ 0 if {LFC}_{ij} = 0\end{matrix}\right.$ (2)

Hierarchical clustering was applied to determine the natural grouping of gene hit scores, with K-means clustering used to define the cutoff between clusters, resulting in six optimal clusters. The cluster with the lowest mean score was excluded, and its maximum value was set as the cutoff threshold for filtering genes. The clustering analysis is illustrated in FIG 7 to 10. The final scores were primarily influenced by the number of comparisons in which a gene appeared, an intentional approach to ensure that genes consistently identified across multiple comparisons were prioritized as true positives in the screening. Genes scoring above the cutoff threshold were designated as top hits, indicating their potential role in enhancing cell growth.

A core set of 235 target genes (and corresponding sgRNA-sets, SEQ ID NO: 236 to 1636) was identified for engineering novel, faster-growing CHO cells, in addition to 2,419 essential genes whose knockout hindered cell survival or reduced growth rates. The target genes were divided into five clusters, ranked in descending order as follows: 4, 1, 5, 3, and 2, including predicted genes and genes that were identified using previous historical filtering criteria based on a false discovery rate (FDR)-adjusted p-value threshold of 0.05. These genes, which appeared in at least one comparison without formal score or filtering, were assigned to cluster 6.

A gene-phenotype relationship was successfully established, confirming that all identified genes were relevant knockout targets with experimentally validated effects on CHO cell growth in the whole-genome KO screen. These genes hold significant potential for developing engineered CHO cell lines with enhanced growth rates, and are listed in **Table 6,** with a preferred subset of identified genes listed in **Table 7.** Furthermore, a crucial aspect of this approach is that the USP (upstream processing) performance, including factors such as titer and viability, is not significantly impaired by the removal of these genes.

**Table 6**

| **Gene Name** | **Gene Product** | **Uniprot ID** | **Description_v2** | **F.Score. pos** | **Cluster** |
|---|---|---|---|---|---|
| **CgPICR_016292** | RALGAPA1 (Fragment) | A0A3L7HH93 | Rap/ran-GAP | 6.69 | 4 |
| **STK25** | STK25 | ADA3L7ICA2 | Protein tyrosine kinase | 6.62 | 4 |
| **CgPICR_021196** | Uncharacterized protein (Fragment) | A0A3L7GZS1 | zinc finger protein | 5.84 | 4 |
| **SP110** | SP110 | A0A3L7IC05 | innate immune response | 5.62 | 4 |
| **CAPN1** | Calcium-activated neutral proteinase 1 | A0A3L7HVZ2 | self proteolysis | 4.65 | 1 |
| **BLOC1S6** | Biogenesis of lysosome-related organelles complex 1 subunit 6 | G3HMD4 | Component of the BLOC-1 complex. a complex that is required for normal biogenesis of lysosome-related organelles (LRO). such as platelet dense granules and melanosomes. In concert with the AP-3 complex. the BLOC-1 complex is required to target membrane protein cargos into vesicles assembled at cell bodies for delivery into neurites and nerve terminals. The BLOC-1 complex. in association with SNARE proteins. is also proposed to be involved in neurite extension. May play a role in intracellular vesicle trafficking. particularly in the vesicle-docking and fusion process | 4.54 | 1 |
| **CgPICR_010567** | HAGHL (Fragment) | A0A3L7HAS4 | Hydroxyacylglutathione hydrolase C-terminus | 4.51 | 1 |
| **GTF2H1** | GTF2H1 (Fragment) | A0A3L7HUN1 | General transcription factor IIH | 4.48 | 1 |
| **SLC36A2** | SLC36A2 | A0A3L7GPH0 | regulation of proline transport | 4.32 | 1 |
| **CHRM3** | Muscarinic acetylcholine receptor | G312C8 | The muscarinic acetylcholine receptor mediates various cellular responses. including inhibition of adenylate cyclase. breakdown of phosphoinositides and modulation of potassium channels through the action of G proteins | 4.00 | 1 |
| **NA** | hypothetical protein | A0A6J0DQI4 | Breast carcinoma amplified sequence 3 | 3.86 | 1 |
| **UNC5B** | Netrin receptor UNC5 | A0A3L7ILW5 | netrin receptor activity | 3.83 | 1 |
| **EIF3E** | EIF3E (Fragment) | A0A3L7IPN2 | Component of the eukaryotic translation initiation factor 3 (eIF-3) complex. which is required for several steps in the initiation of protein synthesis. The eIF-3 complex associates with the 40S ribosome and facilitates the recruitment of eIF-1. eIF-1A. eIF-2 GTP methionyl-tRNAi and eIF-5 to form the 43S pre-initiation complex (43S PIC). The eIF-3 complex stimulates mRNA recruitment to the 43S PIC and scanning of the | 3.74 | 1 |
| | | | mRNA for AUG recognition. The eIF-3 complex is also required for disassembly and recycling of post-termination ribosomal complexes and subsequently prevents premature joining of the 40S and 60S ribosomal subunits prior to initiation. The eIF-3 complex specifically targets and initiates translation of a subset of mRNAs involved in cell proliferation. including cell cycling. differentiation and apoptosis. and uses different modes of RNA stem-loop binding to exert either translational activation or repression. Required for nonsense-mediated mRNA decay (NMD) | | |
| **APPBP2** | APPBP2 (Fragment) | A0A3L7GN32 | Amyloid beta precursor protein (cytoplasmic tail) binding protein 2 | 3.74 | 1 |
| **HOXC6** | HOXC6 | G3HV25 | Homeobox protein Hox-C6 | 3.68 | 1 |
| **MORC4** | MORC4 (Fragment) | A0A3L7H299 | CW-type Zinc Finger | 3.60 | 1 |
| **TARS** | Threonyl-tRNA synthetase | A0A3L7I6R1 | threonine-tRNA ligase activity | 3.59 | 1 |
| **LRRC19** | LRRC19 | A0A3L7I6Z8 | Leucine-rich repeat family 19 TM domain | 3.57 | 1 |
| **TNS2** | TNS2 | A0A3L7IB82 | C1 domain-containing phosphatase | 3.56 | 1 |
| **NA** | hypothetical protein | NA | NA | 3.53 | 1 |
| **SET** | SET | A0A3L7IBU0 | Belongs to the nucleosome assembly protein (NAP) family | 3.52 | 1 |
| **CgPICR_018086** | Uncharacterized protein | A0A3L7I1A0 | Domain of unknown function (DUF4602) | 3.49 | 1 |
| **ASB15** | ASB15 | A0A3L7ILK5 | SOCS_box | 3.48 | 1 |
| **CgPICR_012913** | WISP2 (Fragment) | A0A3L7HD10 | insulin-like growth factor binding | 3.41 | 1 |
| **H2AFJ** | Histone H2A | A0A3L7H6P6 | C-terminus of histone H2A | 3.37 | 1 |
| **ME2** | Malic enzyme | A0A3L7IAF1 | Malic enzyme. NAD binding domain | 3.27 | 1 |
| **FcER1G** | Fc receptor gamma-chain | A0A3L7HIQ7 | High affinity immunoglobulin epsilon receptor subunit gamma | 3.20 | 5 |
| **JADE3** | JADE3 (Fragment) | G3H3Y3 | Jade family PHD finger | 3.18 | 5 |
| **TBX18** | TBX18 | A0A3L7HUM8 | Domain first found in the mice T locus (Brachyury) protein | 3.18 | 5 |
| **CHIC2** | CHIC2 | A0A3L7IJL0 | Golgin subfamily A member 7/ERF4 family | 3.11 | 5 |
| **RXRB** | RXRB | A0A3L7ILA5 | Retinoic acid receptor RXR-beta | 3.10 | 5 |
| **MSANTD4** | Myb/SANT-like DNA-binding domain-containing protein 4 | G3HPN2 | Myb/SANT-like DNA-binding domain | 3.06 | 5 |
| **RHOXF2B** | RHOXF2B | A0A3L7H4P8 | homeobox | 3.03 | 5 |
| **CgPICR_021213** | ZNF420 (Fragment) | A0A3L7GZV3 | nucleic acid-templated transcription | 3.01 | 5 |
| **PANK4** | 4'-phosphopantetheine phosphatase | ADA3L7I9H5 | pantothenate kinase activity | 3.01 | 5 |
| **OLFR1013** | OLFR1013 | A0A3L7HFR8 | Olfactory receptor | 2.98 | 5 |
| **NDRG4** | NDRG4 | A0A3L7HZW8 | N-myc downstream regulated 1 | 2.91 | 5 |
| **TSPAN32** | TSPAN32 | A0A3L7HWB9 | Belongs to the tetraspanin (TM4SF) family | 2.89 | 5 |
| **RPL35** | 60S ribosomal protein L35 | G3HZI1 | 60S ribosomal protein | 2.89 | 5 |
| **SLC12A2** | SLC12A2 | A0A3L7I743 | Amino acid permease N-terminal | 2.84 | 5 |
| **CEMIP** | Hyaluronoglucosaminidase | A0A3L7HZU3 | Cell migration-inducing and hyaluronan-binding protein | 2.81 | 5 |
| **NA** | hypothetical protein | Q8C672 | NA | 2.81 | 5 |
| **SET** | SET | A0A3L7HDL6 | Belongs to the nucleosome assembly protein (NAP) family | 2.78 | 5 |
| **NA** | hypothetical protein | Q63305 | Reverse transcriptase (RNA-dependent DNA polymerase) | 2.77 | 5 |
| **AHDC1** | AHDC1 | A0A3L7I8C5 | NA | 2.65 | 5 |
| **UBALD1** | UBALD1 | A0A3L7H3J0 | UBA-like domain-containing protein 1 | 2.65 | 5 |
| **NADK2** | NADK2 | A0A3L7I5U4 | NAD kinase | 2.65 | 5 |
| **NA** | hypothetical protein | A0A3Q0CLR7 | Xenotropic and polytropic retrovirus receptor 1 | 2.56 | 5 |
| **SPARC** | Osteonectin (Fragment) | A0A3L7GMY7 | SPARC isoform X1 | 2.51 | 5 |
| **NA** | hypothetical protein | Q63305 | Reverse transcriptase (RNA-dependent DNA polymerase) | 2.51 | 5 |
| **MPST** | Sulfurtransferase | A0A3L7I2F1 | 3-mercaptopyruvate sulfurtransferase | 2.50 | 5 |
| **PTAR1** | PTAR1 | A0A3L7I0I1 | Protein prenyltransferase alpha subunit | 2.48 | 5 |
| **CCDC32** | CCDC32 | A0A3L7HEY6 | protein C15orf57 homolog | 2.47 | 5 |
| **BCL2L12** | BCL2L12 | A0A3L7HFV1 | Bcl-2-like protein 12 | 2.47 | 5 |
| **FAM83A** | FAM83A | A0A3L7IPH8 | Protein of unknown function (DUF1669) | 2.47 | 5 |
| **SSH1** | SSH1 | A0A3L7HP32 | Protein phosphatase Slingshot homolog 3 | 2.46 | 5 |
| **DCP1B** | DCP1B | A0A3L7H8M0 | mRNA-decapping enzyme 1B | 2.42 | 5 |
| **VEGFC** | VEGFC | A0A3L7IIZ7 | Vascular endothelial growth factor C | 2.39 | 5 |
| **PML** | PML | A0A3L7HNH0 | Protein PML isoform | 2.39 | 5 |
| **CYP2C18** | CYP2C18 | A0A3L7HYJ8 | aromatase activity | 2.36 | 5 |
| **TCOF1** | TCOF1 | A0A3L7GUI8 | Treacle protein | 2.34 | 5 |
| **NA** | hypothetical protein | NA | NA | 2.29 | 5 |
| **IF1204** | IFI204 | A0A3L7HKU7 | HIN-200/IF120x domain | 2.21 | 5 |
| **MLLT6** | MLLT6 | A0A3L7HBR9 | PHD-zinc-finger like domain | 2.19 | 5 |
| **SH3BGRL** | SH3BGRL | A0A3L7H2C5 | SH3 domain-binding glutamic acid-rich-like protein-like | 213 | 5 |
| **ADAMTS15** | ADAMTS15 | A0A3L7HTF7 | A disintegrin and metalloproteinase with thrombospondin motifs 15 | 2.06 | 3 |
| **NA** | hypothetical protein | NA | NA | 2.04 | 3 |
| **SNX25** | SNX25 | A0A3L7IIR6 | Domain associated with PX domains | 2.02 | 3 |
| **DRC3** | DRC3 | A0A3L7GMT4 | Leucine-rich repeat-containing protein 48 | 1.94 | 3 |
| **PTBP1** | PTBP1 | A0A3L7GVZ1 | regulation of secretory granule organization | 1.93 | 3 |
| **KLHL22** | Kelch-like protein 22 | A0A3L7HT84 | Kelch-like protein 22 | 1.87 | 3 |
| **HLX** | HLX | A0A3L7HJV4 | negative regulation of T-helper 2 cell differentiation | 1.85 | 3 |
| **SRD5A2** | SRD5A2 | A0A3L7H9K6 | 3-oxo-5-alpha-steroid 4-dehydrogenase 1 | 1.83 | 3 |
| **LRRK2** | Non-specific serine/threonine protein kinase (Fragment) | A0A3L7IAL4 | Leucine-rich repeat serine threonine-protein kinase 2 | 1.79 | 3 |
| **TCEB2** | TCEB2 | A0A3L7I4F4 | transcription elongation factor b | 1.75 | 3 |
| **ZEB2** | ZEB2 | A0A3L7HGS6 | Zinc finger E-box-binding homeobox 1 | 1.74 | 3 |
| **SWI5** | DNA repair protein SWI5 homolog (Fragment) | A0A3L7HCM7 | DNA repair protein SWI5 homolog | 1.73 | 3 |
| **TMEM79** | TMEM79 | G3HG88 | cuticle development | 1.72 | 3 |
| **CDH1** | Cadherin-1 | A0A3L7I2U6 | Cadherins are calcium-dependent cell adhesion proteins | 1.70 | 3 |
| **EIF1** | EIF1 | A0A3L7HC78 | eukaryotic translation initiation factor | 1.69 | 3 |
| **MIA3** | MIA3 | A0A3L7HI86 | Melanoma inhibitory activity | 1.61 | 3 |
| **CgPICR_007239** | EXTL1 | A0A3L7IA81 | N-acetylglucosaminyl-proteoglycan 4-beta-glucuronosyltransferase activity | 1.59 | 3 |
| **SLC36A4** | SLC36A4 | A0A3L7HTK8 | Proton-coupled amino acid transporter 4 | 1.59 | 3 |
| **CLEC4F** | CLEC4F | A0A3L7GUG2 | carbohydrate binding | 1.55 | 3 |
| **CgPICR_022463** | ZCCHC11 (Fragment) | A0A3L7GX88 | Terminal uridylyltransferase 4 | 1.54 | 3 |
| **CRLF2** | CRLF2 | A0A3L7GWJ0 | Cytokine receptor-like factor | 1.53 | 3 |
| **CgPICR_007622** | Phosphoenolpyruvate carboxykinase (GTP) (Fragment) | A0A3L7GR08 | Pfam:PEPCK | 1.52 | 3 |
| **LHX6** | LHX6 | A0A3L7HCB0 | LIM homeobox protein Lhx6 | 1.52 | 3 |
| **RPL7** | RPL7 | A0A3L7I8Z7 | 60S ribosomal protein | 1.51 | 3 |
| **HOXB3** | HOXB3 | A0A3L7HBU7 | Domain of unknown function (DUF4074) | 1.51 | 3 |
| **PSAP** | PSAP | A0A3L7IP82 | glycolipid transport | 1.50 | 3 |
| **LATS2** | LATS2 | G3H8I4 | Serine threonine-protein kinase LATS2 | 1.50 | 3 |
| **CgPICR_021223** | ZNF420 (Fragment) | A0A3L7GZW3 | Zinc finger protein | 1.50 | 3 |
| **NA** | hypothetical protein | G3H710 | NA | 1.50 | 3 |
| **FCGBP** | FCGBP | A0A3L7H806 | VWC_def | 1.50 | 3 |
| **RPL35** | RPL35 | A0A3L7HWQ2 | 60S ribosomal protein | 1.49 | 3 |
| **STK17B** | STK17B | A0A3L7ID00 | Serine/Threonine protein kinases. catalytic domain | 1.49 | 3 |
| **NA** | hypothetical protein | NA | NA | 1.49 | 3 |
| **FGF13** | Fibroblast growth factor (Fragment) | A0A3L7H1Y1 | fibroblast growth factor 12 | 1.49 | 3 |
| **ZDHHC20** | Palmitoyltransferase | ADA3L7GQTS | palmitoyltransferase ZDHHC20 | 1.48 | 3 |
| **SULT3A1** | Sulfotransferase | A0A3L7I4J4 | amine sulfotransferase activity | 1.48 | 3 |
| **FBXO30** | F-box only protein 30 | G3HXS5 | ubiquitin-like protein ligase activity | 1.47 | 3 |
| **AKAP3** | AKAP3 | A0A3L7H8T3 | anchor protein 3 | 1.44 | 3 |
| **ANGPT2** | Angiopoietin-2 | A0A3L7IK35 | Binds to TEK TIE2. competing for the ANGPT1 binding site. and modulating ANGPT1 signaling. Can induce tyrosine phosphorylation of TEK TIE2 in the absence of ANGPT1. In the absence of angiogenic inducers. such as VEGF. ANGPT2-mediated loosening | 1.42 | 3 |
| | | | of cell-matrix contacts may induce endothelial cell apoptosis with consequent vascular regression. In concert with VEGF. it may facilitate endothelial cell migration and proliferation. thus serving as a permissive angiogenic signal (By similarity) | | |
| **RIMS1** | RIMS1 | A0A3L7IGQ5 | spontaneous neurotransmitter secretion | 1.42 | 3 |
| **TECPR2** | TECPR2 | A0A3L7HLA9 | Propeller | 1.42 | 3 |
| **FAM98B** | FAM98B | A0A3L7HEW8 | Family with sequence similarity 98 member B | 1.39 | 3 |
| **CCNG2** | CCNG2 | ADA3L7INE3 | Cyclin. N-terminal domain | 1.39 | 3 |
| **PDE1C** | Phosphodiesterase | A0A3L7H700 | Calcium calmodulin-dependent 3'.5'-cyclic nucleotide phosphodiesterase 1C | 1.34 | 3 |
| **NA** | hypothetical protein | A0A061IHY9 | Arb2 domain | 1.32 | 3 |
| **CLEC2H** | CLEC2H (Fragment) | A0A3L7HAZ9 | C-type lectin domain family 2 member | 1.31 | 3 |
| **MUC19** | MUC19 | A0A3L7ICC8 | C-terminal cystine knot-like domain (CTCK) | 1.31 | 3 |
| **KIDINS220** | KIDINS220 | A0A3L7HD36 | KAP family P-loop domain | 1.30 | 3 |
| **BACH1** | BACH1 | A0A3L7HQE0 | Transcription regulator protein BACH2 | 1.28 | 3 |
| **NA** | hypothetical protein | NA | NA | 1.26 | 3 |
| **ZNF420** | ZNF420 (Fragment) | A0A3L7H5Z5 | C2H2-type zinc finger | 1.26 | 3 |
| **CHD7** | CHD7 | A0A3L7IBQ0 | chromodomain-helicase-DNA-binding protein 7 | 1.26 | 3 |
| **KLHL23** | KLHL23 | G3GU91 | Kelch-like protein 23 | 1.24 | 3 |
| **PCDHGC5** | PCDHGC5 | A0A3L7IA53 | Protocadherin | 1.24 | 3 |
| **DPP9** | DPP9 | A0A3L7HL01 | X-Pro dipeptidyl-peptidase (S15 family) | 1.14 | 2 |
| **METAP1** | Methionine aminopeptidase | A0A3L7ID05 | Metallopeptidase family M24 | 1.09 | 2 |
| **NFS1** | NFS1 | A0A3L7I997 | high mobility group | 1.09 | 2 |
| **AREL1** | AREL1 | A0A3L7HJW8 | E3 ubiquitin protein ligase 1 | 1.09 | 2 |
| **SLC24A1** | SLC24A1 (Fragment) | A0A3L7HTI0 | Sodium/calcium exchanger protein | 1.09 | 2 |
| **PSMD2** | 26S proteasome non-ATPase regulatory subunit 2 | A0A3L7HT01 | proteasome (prosome. macropain) 26S subunit. non-ATPase. 2 | 1.09 | 2 |
| **CgPICR_012677** | CXXC motif containing zinc binding protein (Fragment) | A0A3L7I411 | Eukaryotic protein of unknown function (DUF866) | 1.09 | 2 |
| **TOMT** | Catechol O-methyltransferase 2 | A0A061IIA7 | Transmembrane O-methyltransferase | 1.09 | 2 |
| **CgPICR_004773** | RBM26 (Fragment) | A0A3L7IF80 | RNA-binding protein 26 | 1.09 | 2 |
| **NPM2** | NPM2 | A0A3L7GQF2 | Nucleoplasmin/nucleophosmin domain | 1.09 | 2 |
| **OR51I2** | OR5112 | A0A3L7HXM0 | Olfactory receptor | 1.09 | 2 |
| **TRHR** | Thyroliberin receptor | A0A3L7HU01 | Belongs to the G-protein coupled receptor 1 family | 1.09 | 2 |
| **MAP3K7CL** | MAP3K7CL | A0A3L7HR29 | MAP3K7 C-terminal-like | 1.09 | 2 |
| **SLC7A14** | SLC7A14 | A0A3L7IHC7 | C-terminus of AA_permease | 1.09 | 2 |
| **NWD2** | NWD2 | A0A3L7IJ74 | Leucine-rich repeat and WD repeat-containing protein KIAA1239 | 1.09 | 2 |
| **TCP11L1** | TCP11L1 | A0A3L7HHG6 | T-complex protein 11 | 1.09 | 2 |
| **RPL23A** | RPL23A | A0A3L7GLT3 | ribosomal large subunit assembly | 1.09 | 2 |
| **PHF7** | PHF7 | A0A3L7GUY6 | PHD finger protein 7 | 1.09 | 2 |
| **OSTC** | Oligosaccharyltransferase complex subunit | G31H78 | Oligosaccharyltransferase complex subunit | 1.09 | 2 |
| **CgPICR_022530** | Ig-like domain-containing protein | ADA3L7GYT4 | Immunoglobulin V-Type | 1.09 | 2 |
| **APOPT1** | APOPT1 | A0A3L7H873 | positive regulation of release of cytochrome c from mitochondria | 1.09 | 2 |
| **BRD9** | BRD9 | A0A3L7I9B2 | Bromodomain-containing protein 9 | 1.09 | 2 |
| **TCN2** | TCN2 | A0A3L7IR32 | cobalamin transport | 1.09 | 2 |
| **CgPICR_018207** | NCOA7 (Fragment) | ADA3L7I9U5 | Nuclear receptor coactivator | 1.09 | 2 |
| **ZNF764** | ZNF764 | A0A3L7I1N1 | krueppel associated box | 1.09 | 2 |
| **RPS19** | 40S ribosomal protein S19 | A0A061HVT8 | ribosomal protein | 1.09 | 2 |
| **TPRG1L** | TPRG1L | G3H0R6 | identical protein binding | 1.07 | 2 |
| **CgPICR_000061** | Mitogen-activated protein kinase (Fragment) | A0A3L7IAR0 | Mitogen-activated protein kinase 11 | 1.04 | 2 |
| **MED11** | Mediator of RNA polymerase II transcription subunit 11 | G3GYB2 | Mediator complex protein | 0.84 | 2 |
| **ST5** | ST5 | A0A3L7HUE8 | Suppression of tumorigenicity 5 | 0.84 | 2 |
| **DNAJB14** | DNAJB14 | A0A3L7IFA4 | Domain of unknown function (DUF1977) | 0.84 | 2 |
| **UNC80** | UNC80 (Fragment) | A0A3L7IBK8 | Cation channel complex component UNC80 | 0.84 | 2 |
| **NA** | hypothetical protein | A0A6P5R444 | secretion activator | 0.84 | 2 |
| **BRCC3** | Lys-63-specific deubiquitinase | A0A3L7H338 | histone H2A K63-linked deubiquitination | 0.84 | 2 |
| **SLC25A25** | SLC25A25 | A0A3L7HCH0 | Belongs to the mitochondrial carrier (TC 2.A.29) family | 0.84 | 2 |
| **CgPICR_002615** | Oxysterol-binding protein (Fragment) | ADA3L7IHX9 | Oxysterol-binding protein-related protein | 0.84 | 2 |
| **DDIT4** | DDIT4 | ADA3L7IM37 | DNA damage-inducible transcript 4 protein | 0.84 | 2 |
| **NA** | hypothetical protein | NA | NA | 0.84 | 2 |
| **TENM4** | TENM4 | ADA3L7HXF8 | GHH signature containing HNH/Endo VII superfamily nuclease toxin | 0.84 | 2 |
| **TSKS** | TSKS | A0A3L7HFV9 | serine kinase substrate | 0.84 | 2 |
| **ZNF865** | ZNF865 | A0A3L7H734 | Zinc finger protein | 0.84 | 2 |
| **CCDC34** | CCDC34 | G316E1 | coiled-coil domain-containing protein 34 | 0.84 | 2 |
| **WDR62** | WDR62 | A0A3L7H4L4 | WD domain. G-beta repeat | 0.84 | 2 |
| **MBD1** | MBD1 | A0A3L7IA99 | domain protein 1 | 0.84 | 2 |
| **PNPLA4** | PNPLA4 | A0A3L7GXY9 | Patatin-like phospholipase | 0.82 | 2 |
| **SCN1A** | Sodium channel protein | A0A3L7HGG0 | Mediates the voltage-dependent sodium ion permeability of excitable membranes | 0.78 | 2 |
| **CgPICR_013823** | Gamma-glutamyl carboxylase (Fragment) | A0A3L7H7E7 | gamma-glutamyl carboxylase activity | 0.76 | 2 |
| **UBE2Q1** | UBE2Q1 | A0A3L7IGG5 | Ubiquitin-conjugating enzyme | 0.72 | 2 |
| **CgPICR_011804** | ALDH8A1 (Fragment) | A0A3L7I7C4 | Aldehyde dehydrogenase family | 0.72 | 2 |
| **HMGB1** | High mobility group protein 1 | A0A3L7HQA2 | HORMA domain | 0.71 | 2 |
| **MXD3** | MXD3 | A0A3L7HUU4 | protein dimerization activity | 0.70 | 2 |
| **SETBP1** | SETBP1 | A0A3L7ICU7 | SET-binding protein | 0.70 | 2 |
| **ICA** | Beta-1 metal-binding globulin | A0A3L7HNU8 | Transferrin | 0.70 | 2 |
| **SMAD1** | SMAD1 (Fragment) | A0A3L7GVH1 | embryonic pattern specification | 0.70 | 2 |
| **OR7G1** | Olfactory receptor | A0A3L7HT87 | Olfactory receptor 7G2-like isoform X2 | 0.68 | 2 |
| **NA** | hypothetical protein | A0A1U7QEJ4 | Partitioning defective 3 homolog B | 0.66 | 2 |
| **PRRT3** | PRRT3 | A0A3L7H7I2 | Proline-rich transmembrane protein 3 | 0.65 | 2 |
| **MID1IP1** | MID11P1 | A0A3L7H1W2 | Mid1-interacting protein 1 | 0.64 | 2 |
| **ZNF462** | ZNF462 (Fragment) | A0A3L7I332 | Zinc finger protein 462 | 0.62 | 2 |
| **CRTC2** | CRTC2 | A0A3L7IGI5 | transcription coactivator 3 | 0.61 | 2 |
| **ENTPD7** | ENTPD7 | A0A3L7HY78 | GDA1/CD39 (nucleoside phosphatase) family | 0.61 | 2 |
| **CNNM3** | CNNM3 (Fragment) | A0A3L7INQ5 | CBS domain | 0.60 | 2 |
| **NA** | hypothetical protein | A0A6I9LQM5 | regulation of erythrocyte clearance | 0.60 | 2 |
| **PKP4** | PKP4 | A0A3L7HGM0 | Armadillo/beta-catenin-like repeat | 0.60 | 2 |
| **GRIA2** | Glutamate receptor | A0A3L7IFY0 | Eukaryotic homologues of bacterial periplasmic substrate binding proteins | 0.60 | 2 |
| **NTSR2** | NTSR2 | A0A3L7HB91 | G-protein coupled receptor | 0.60 | 2 |
| **CCDC106** | CCDC106 | A0A3L7H5B0 | Coiled-coil domain-containing protein 106 | 0.59 | 2 |
| **RPS11** | 40S ribosomal protein S11 | G31004 | rRNA binding | 0.59 | 2 |
| **XPNPEP1** | XPNPEP1 | A0A3L7HZN3 | bradykinin catabolic process | 0.59 | 2 |
| **NA** | hypothetical protein | NA | NA | 0.59 | 2 |
| **LMAN2L** | LMAN2L | A0A3L7INB6 | VIP36-like protein isoform X1 | 0.58 | 2 |
| **SERPINI2** | SERPINI2 | A0A3L7IJU3 | SERine Proteinase INhibitors | 0.58 | 2 |
| **GLB1L2** | Beta-galactosidase | A0A3L7HRR9 | Beta-galactosidase-1-like protein | 0.58 | 2 |
| **TLE3** | TLE3 | A0A3L7HNI8 | Transducin-like enhancer | 0.57 | 2 |
| **ENV** | ENV (Fragment) | A0A3L7ILG3 | ENV polyprotein (coat polyprotein) | 0.57 | 2 |
| **SPATA31A6** | SPATA31A6 | A0A3L7HVL5 | FAM75 family | 0.57 | 2 |
| **IGH-VJ558** | IGH-VJ558 | A0A3L7GXZ1 | Immunoglobulin V-Type | 0.57 | 2 |
| **GRAP** | GRAP | ADA3L7GMQ3 | GRB2-related adapter protein | 0.56 | 2 |
| **COL4A2** | COL4A2 | A0A3L7IPF5 | Collagen alpha-2(IV) chain | 0.55 | 2 |
| **ARL4D** | ADP-ribosylation factor-like protein 4D | G3INU1 | ADP-ribosylation factor-like | 0.38 | 6 |
| **CgPICR_006791** | Uncharacterized protein | A0A3L7IJM4 | T-cell receptor | 0.39 | 6 |
| **DEPTOR** | DEPTOR | A0A3L7IPG2 | Belongs to the universal ribosomal protein uL23 family | 0.39 | 6 |
| **DISP1** | DISP1 (Fragment) | A0A3L7HKM4 | Dispatched homolog 1 | 0.38 | 6 |
| **FXYD7** | FXYD domain-containing ion transport regulator | A0A3L7H4V0 | sodium channel regulator activity | 0.38 | 6 |
| **GRK6** | G protein-coupled receptor kinase | A0A3L7HWI8 | G protein-coupled receptor kinase | 0.39 | 6 |
| **MACF1** | MACF1 | A0A3L7I7Y9 | Microtubule-actin cross-linking factor 1 | 0.38 | 6 |
| **MORF4L2** | Mortality factor 4-like protein 2 | A0A3L7H233 | Mortality factor 4-like protein 2 | 0.39 | 6 |
| **PAG1** | PAG1 | A0A3L7I918 | Phosphoprotein associated with glycosphingolipid-enriched microdomains 1 | 0.38 | 6 |
| **RILPL1** | RILPL1 | A0A3L7HR56 | RILP-like protein 1 | 0.39 | 6 |
| **SIMC1** | SIMC1 | A0A3L7HVK6 | spliceosomal complex assembly | 0.38 | 6 |
| **STUB1** | STUB1 | A0A3L7HCC4 | STIP1 homology and | 0.38 | 6 |
| **SYTL1** | SYTL1 | A0A3L7I909 | Synaptotagmin-like protein 1 | 0.38 | 6 |
| **TMEM55A** | Phosphatidylinositol-4%2C5-bisphosphate 4-phosphatase | A0A3L7I3P7 | Transmembrane protein 55A | 0.39 | 6 |
| **TRIM2** | TRIM2 | A0A3L7IG82 | regulation of neuron apoptotic process | 0.38 | 6 |
| **VMN2R116** | VMN2R116 | A0A3L7H160 | 7 transmembrane sweet-taste receptor of 3 GCPR | 0.39 | 6 |
| **FOXO3** | FOXO3 | G3GXK7 | KIX-binding domain of forkhead box O. CR2 | 0.186 | 6 |
| **KDM5C** | KDM5A | G3H882 | PLU-1-like protein | 0.186 | 6 |
| **CTNNA1** | CTNNA3 | G3H9H8 | Catenin alpha-3 | 0.186 | 6 |
| **ARID1B** | ARID1B | G3HFW3 | SWI/SNF-like complex subunit BAF250/Osa | 0.257 | 6 |
| **LZTR1** | LZTR1 | G3HG01 | Leucine-zipper-like transcriptional regulator 1 | 0.257 | 6 |
| **GPC3** | GPC3 | G3HGH9 | Cell surface proteoglycan that bears heparan sulfate | 0.186 | 6 |
| **TRAF7** | TRAF7 | G3HHB5 | E3 ubiquitin-protein ligase TRAF7 | 0.257 | 6 |
| **HNF1A** | HNF1A | G3HI63 | Hepatocyte nuclear factor 1-alpha | 0.257 | 6 |
| **PTPRK** | PTPRM | G3HIV1 | Protein tyrosine phosphatase. catalytic domain | 0.384 | 6 |
| **CTNNA1** | CTNNA3 | G3HN58 | Catenin alpha-3 | 0.186 | 6 |
| **GATA1** | GATA1 | G3HND3 | basophil differentiation | 0.257 | 6 |
| **RPL5** | RPL5 | G3HNJ6 | positive regulation of isoleucine-tRNA lipase activity | 0.257 | 6 |
| **NF2** | NF2 | G3HP12 | Ezrin/radixin/moesin family | 0.186 | 6 |
| **RPL5** | RPL5 | G3HVH8 | positive regulation of isoleucine-tRNA ligase activity | 0.384 | 6 |
| **SMARCD1** | SMARCD1 | G3HX65 | regulator of chromatin subfamily D member 1 | 0.186 | 6 |
| **EBF3** | EBF2 | G3HY76 | C2H2 zinc finger domain binding | 0.257 | 6 |
| **CDS1** | CDS1 | G315J8 | Phosphatidate cytidylyltransferase 1 | 0.257 | 6 |
| **CASP9** | CASP9 | G31954 | Caspase recruitment domain | 0.257 | 6 |
| **ARHGAP35** | ARHGAP5 | G3ICT9 | rho GTPase-activating protein | 0.257 | 6 |
| **RXRA** | RXRA | G3IDY0 | Retinoic acid receptor RXR-alpha | 0.186 | 6 |
| **FAH** | FAH | A0A3L7HX59 | fumarylacetoacetase activity | 0.257 | 6 |

**Table 7**

| **Gene Name** | **Gene Product** | **Uniprot ID** | **Description_v2** | **F.Score. pos** | **Cluster** |
|---|---|---|---|---|---|
| **CgPICR_016292** | RALGAPA1 (Fragment) | A0A3L7HH93 | Rap/ran-GAP | 6.69 | 4 |
| **CgPICR_021196** | Uncharacterized protein (Fragment) | A0A3L7GZS1 | zinc finger protein | 5.84 | 4 |
| **SP110** | SP110 | A0A3L7IC05 | innate immune response | 5.62 | 4 |
| **CAPN1** | Calcium-activated neutral proteinase 1 | A0A3L7HVZ2 | self proteolysis | 4.65 | 1 |
| **BLOC1S6** | Biogenesis of lysosome-related organelles complex 1 subunit 6 | G3HMD4 | Component of the BLOC-1 complex. a complex that is required for normal biogenesis of lysosome-related organelles (LRO). such as platelet dense granules and melanosomes. In concert with the AP-3 complex. the BLOC-1 complex is required to target membrane protein cargos into vesicles assembled at cell bodies for delivery into neurites and nerve terminals. | 4.54 | 1 |
| | | | The BLOC-1 complex. in association with SNARE proteins. is also proposed to be involved in neurite extension. May play a role in intracellular vesicle trafficking. particularly in the vesicle-docking and fusion process | | |
| **GTF2H1** | GTF2H1 (Fragment) | A0A3L7HUN1 | General transcription factor IIH | 4.48 | 1 |
| **SLC36A2** | SLC36A2 | A0A3L7GPH0 | regulation of proline transport | 4.32 | 1 |
| **CHRM3** | Muscarinic acetylcholine receptor | G312C8 | The muscarinic acetylcholine receptor mediates various cellular responses. including inhibition of adenylate cyclase. breakdown of phosphoinositides and modulation of potassium channels through the action of G proteins | 4.00 | 1 |
| **NA** | hypothetical protein | A0A6J0DQI4 | Breast carcinoma amplified sequence 3 | 3.86 | 1 |
| **UNC5B** | Netrin receptor UNC5 | A0A3L7ILW5 | netrin receptor activity | 3.83 | 1 |
| **EIF3E** | EIF3E (Fragment) | ADA3L7IPN2 | Component of the eukaryotic translation initiation factor 3 (eIF-3) complex. which is required for several steps in the initiation of protein synthesis. The eIF-3 complex associates with the 40S ribosome and facilitates the recruitment of eIF-1. eIF-1A. eIF-2 GTP methionyl-tRNAi and eIF-5 to form the 43S pre-initiation complex (43S PIC). The eIF-3 complex stimulates mRNA recruitment to the 43S PIC and scanning of the mRNA for AUG recognition. | 3.74 | 1 |
| | | | The eIF-3 complex is also required for disassembly and recycling of post-termination | | |
| | | | ribosomal complexes and subsequently prevents premature joining of the 40S and 60S ribosomal subunits prior to initiation. The eIF-3 complex specifically targets and initiates translation of a subset of mRNAs involved in cell proliferation. including cell cycling. differentiation and apoptosis. and uses different modes of RNA stem-loop binding to exert either translational activation or repression. Required for nonsense-mediated mRNA decay (NMD) | | |
| **APPBP2** | APPBP2 (Fragment) | A0A3L7GN32 | Amyloid beta precursor protein (cytoplasmic tail) binding protein 2 | 3.74 | 1 |
| **HOXC6** | HOXC6 | G3HV25 | Homeobox protein Hox-C6 | 3.68 | 1 |
| **MORC4** | MORC4 (Fragment) | A0A3L7H299 | CW-type Zinc Finger | 3.60 | 1 |
| **TARS** | Threonyl-tRNA synthetase | A0A3L7I6R1 | threonine-tRNA ligase activity | 3.59 | 1 |
| **LRRC19** | LRRC19 | A0A3L7I6Z8 | Leucine-rich repeat family 19 TM domain | 3.57 | 1 |
| **TNS2** | TNS2 | A0A3L7IB82 | C1 domain-containing phosphatase | 3.56 | 1 |
| **NA** | hypothetical protein | NA | NA | 3.53 | 1 |
| **SET** | SET | A0A3L7IBU0 | Belongs to the nucleosome assembly protein (NAP) family | 3.52 | 1 |
| **CgPICR_018086** | Uncharacterized protein | A0A3L711A0 | Domain of unknown function (DUF4602) | 3.49 | 1 |
| **ASB15** | ASB15 | A0A3L71LK5 | SOCS_box | 3.48 | 1 |
| **CgPICR_012913** | WISP2 (Fragment) | ADA3L7HD10 | insulin-like growth factor binding | 3.41 | 1 |
| **H2AFJ** | Histone H2A | A0A3L7H6P6 | C-terminus of histone H2A | 3.37 | 1 |
| **ME2** | Malic enzyme | A0A3L71AF1 | Malic enzyme. NAD binding domain | 3.27 | 1 |
| **FCER1G** | Fc receptor gamma-chain | A0A3L7HIQ7 | High affinity immunoglobulin epsilon receptor subunit gamma | 3.20 | 5 |
| **JADE3** | JADE3 (Fragment) | G3H3Y3 | Jade family PHD finger | 3.18 | 5 |
| **TBX18** | TBX18 | A0A3L7HUM8 | Domain first found in the mice T locus (Brachyury) protein | 3.18 | 5 |
| **CHIC2** | CHIC2 | A0A3L71JL0 | Golgin subfamily A member 7/ERF4 family | 3.11 | 5 |
| **RXRB** | RXRB | A0A3L71LA5 | Retinoic acid receptor RXR-beta | 3.10 | 5 |
| **MSANTD4** | Myb/SANT-like DNA-binding domain-containing protein 4 | G3HPN2 | Myb/SANT-like DNA-binding domain | 3.06 | 5 |
| **RHOXF2B** | RHOXF2B | A0A3L7H4P8 | homeobox | 3.03 | 5 |
| **CgPICR_021213** | ZNF420 (Fragment) | A0A3L7GZV3 | nucleic acid-templated transcription | 3.01 | 5 |
| **PANK4** | 4'-phosphopantetheine phosphatase | A0A3L7I9H5 | pantothenate kinase activity | 3.01 | 5 |
| **OLFR1013** | OLFR1013 | A0A3L7HFR8 | Olfactory receptor | 2.98 | 5 |
| **NDRG4** | NDRG4 | A0A3L7HZW8 | N-myc downstream regulated 1 | 2.91 | 5 |
| **TSPAN32** | TSPAN32 | A0A3L7HWB9 | Belongs to the tetraspanin (TM4SF) family | 2.89 | 5 |
| **RPL35** | 60S ribosomal protein L35 | G3HZI1 | 60S ribosomal protein | 2.89 | 5 |
| **SLC12A2** | SLC12A2 | A0A3L71743 | Amino acid permease N-terminal | 2.84 | 5 |
| **CEMIP** | Hyaluronoglucosaminidase | A0A3L7HZU3 | Cell migration-inducing and hyaluronan-binding protein | 2.81 | 5 |
| **NA** | hypothetical protein | Q8C672 | NA | 2.81 | 5 |
| **SET** | SET | A0A3L7HDL6 | Belongs to the nucleosome assembly protein (NAP) family | 2.78 | 5 |
| **NA** | hypothetical protein | Q63305 | Reverse transcriptase (RNA-dependent DNA polymerase) | 2.77 | 5 |
| **AHDC1** | AHDC1 | A0A3L718C5 | NA | 2.65 | 5 |
| **UBALD1** | UBALD1 | A0A3L7H3J0 | UBA-like domain-containing protein 1 | 2.65 | 5 |
| **NADK2** | NADK2 | A0A3L715U4 | NAD kinase | 2.65 | 5 |
| **NA** | hypothetical protein | A0A3Q0CLR7 | Xenotropic and polytropic retrovirus receptor 1 | 2.56 | 5 |
| **SPARC** | Osteonectin (Fragment) | A0A3L7GMY7 | SPARC isoform X1 | 2.51 | 5 |
| **NA** | hypothetical protein | Q63305 | Reverse transcriptase (RNA-dependent DNA polymerase) | 2.51 | 5 |
| **PTAR1** | PTAR1 | A0A3L7I0I1 | Protein prenyltransferase alpha subunit | 2.48 | 5 |
| **CCDC32** | CCDC32 | A0A3L7HEY6 | protein C15orf57 homolog | 2.47 | 5 |
| **BCL2L12** | BCL2L12 | A0A3L7HFV1 | Bcl-2-like protein 12 | 2.47 | 5 |
| **FAM83A** | FAM83A | A0A3L7IPH8 | Protein of unknown function (DUF1669) | 2.47 | 5 |
| **SSH1** | SSH1 | A0A3L7HP32 | Protein phosphatase Slingshot homolog 3 | 2.46 | 5 |
| **DCP1B** | DCP1B | A0A3L7H8M0 | mRNA-decapping enzyme 1B | 2.42 | 5 |
| **VEGFC** | VEGFC | A0A3L7IIZ7 | Vascular endothelial growth factor C | 2.39 | 5 |
| **PML** | PML | A0A3L7HNH0 | Protein PML isoform | 2.39 | 5 |
| **CYP2C18** | CYP2C18 | A0A3L7HYJ8 | aromatase activity | 2.36 | 5 |
| **TCOF1** | TCOF1 | A0A3L7GUI8 | Treacle protein | 2.34 | 5 |
| **NA** | hypothetical protein | NA | NA | 2.29 | 5 |
| **IFI204** | IFI204 | A0A3L7HKU7 | HIN-200/IF120x domain | 2.21 | 5 |
| **MLLT6** | MLLT6 | A0A3L7HBR9 | PHD-zinc-finger like domain | 2.19 | 5 |
| **SH3BGRL** | SH3BGRL | A0A3L7H2C5 | SH3 domain-binding glutamic acid-rich-like protein-like | 2.13 | 5 |
| **ADAMTS15** | ADAMTS15 | A0A3L7HTF7 | A disintegrin and metalloproteinase with thrombospondin motifs 15 | 2.06 | 3 |
| **NA** | hypothetical protein | NA | NA | 2.04 | 3 |
| **SNX25** | SNX25 | A0A3L7IIR6 | Domain associated with PX domains | 2.02 | 3 |
| **DRC3** | DRC3 | A0A3L7GMT4 | Leucine-rich repeat-containing protein 48 | 1.94 | 3 |
| **PTBP1** | PTBP1 | A0A3L7GVZ1 | regulation of secretory granule organization | 1.93 | 3 |
| **KLHL22** | Kelch-like protein 22 | A0A3L7HT84 | Kelch-like protein 22 | 1.87 | 3 |
| **HLX** | HLX | A0A3L7HJV4 | negative regulation of T-helper 2 cell differentiation | 1.85 | 3 |
| **SRD5A2** | SRD5A2 | A0A3L7H9K6 | 3-oxo-5-alpha-steroid 4-dehydrogenase 1 | 1.83 | 3 |
| **LRRK2** | Non-specific serine/threonine protein kinase (Fragment) | A0A3L7IAL4 | Leucine-rich repeat serine threonine-protein kinase 2 | 1.79 | 3 |
| **TCEB2** | TCEB2 | A0A3L7I4F4 | transcription elongation factor b | 1.75 | 3 |
| **ZEB2** | ZEB2 | A0A3L7HGS6 | Zinc finger E-box-binding homeobox 1 | 1.74 | 3 |
| **SWI5** | DNA repair protein SWI5 homolog (Fragment) | A0A3L7HCM7 | DNA repair protein SWI5 homolog | 1.73 | 3 |
| **TMEM79** | TMEM79 | G3HG88 | cuticle development | 1.72 | 3 |
| **CDH1** | Cadherin-1 | A0A3L7I2U6 | Cadherins are calcium-dependent cell adhesion proteins | 1.70 | 3 |
| **EIF1** | EIF1 | A0A3L7HC78 | eukaryotic translation initiation factor | 1.69 | 3 |
| **MIA3** | MIA3 | A0A3L7HI86 | Melanoma inhibitory activity | 1.61 | 3 |
| **CgPICR_007239** | EXTL1 | A0A3L7IA81 | N-acetylglucosaminyl-proteoglycan 4-beta-glucuronosyltransferase activity | 1.59 | 3 |
| **SLC36A4** | SLC36A4 | A0A3L7HTK8 | Proton-coupled amino acid transporter 4 | 1.59 | 3 |
| **CLEC4F** | CLEC4F | A0A3L7GUG2 | carbohydrate binding | 1.55 | 3 |
| **CgPICR_022463** | ZCCHC11 (Fragment) | A0A3L7GX88 | Terminal uridylyltransferase 4 | 1.54 | 3 |
| **CRLF2** | CRLF2 | A0A3L7GWJ0 | Cytokine receptor-like factor | 1.53 | 3 |
| **LHX6** | LHX6 | A0A3L7HCB0 | LIM homeobox protein Lhx6 | 1.52 | 3 |
| **RPL7** | RPL7 | A0A3L7I8Z7 | 60S ribosomal protein | 1.51 | 3 |
| **HOXB3** | HOXB3 | A0A3L7HBU7 | Domain of unknown function (DUF4074) | 1.51 | 3 |
| **PSAP** | PSAP | A0A3L7IP82 | glycolipid transport | 1.50 | 3 |
| **LATS2** | LATS2 | G3H8I4 | Serine threonine-protein kinase LATS2 | 1.50 | 3 |
| **CgPICR_021223** | ZNF420 (Fragment) | A0A3L7GZW3 | Zinc finger protein | 1.50 | 3 |
| **NA** | hypothetical protein | G3H7I0 | NA | 1.50 | 3 |
| **FCGBP** | FCGBP | A0A3L7H806 | VWC_def | 1.50 | 3 |
| **RPL35** | RPL35 | A0A3L7HWQ2 | 60S ribosomal protein | 1.49 | 3 |
| **STK17B** | STK17B | A0A3L7ID00 | Serine/Threonine protein kinases. catalytic domain | 1.49 | 3 |
| **NA** | hypothetical protein | NA | NA | 1.49 | 3 |
| **FGF13** | Fibroblast growth factor (Fragment) | A0A3L7H1Y1 | fibroblast growth factor 12 | 1.49 | 3 |
| **ZDHHC20** | Palmitoyltransferase | A0A3L7GQT5 | palmitoyltransferase ZDHHC20 | 1.48 | 3 |
| **SULT3A1** | Sulfotransferase | A0A3L7I4J4 | amine sulfotransferase activity | 1.48 | 3 |
| **FBXO30** | F-box only protein 30 | G3HXS5 | ubiquitin-like protein ligase activity | 1.47 | 3 |
| **AKAP3** | AKAP3 | A0A3L7H8T3 | anchor protein 3 | 1.44 | 3 |
| **ANGPT2** | Angiopoietin-2 | A0A3L7IK35 | Binds to TEK TIE2. competing for the ANGPT1 binding site. and modulating ANGPT1 signaling. Can induce tyrosine phosphorylation of TEK TIE2 in the absence of ANGPT1. In the absence of angiogenic inducers. such as VEGF. ANGPT2-mediated loosening of cell-matrix contacts may induce endothelial cell apoptosis with consequent vascular regression. In concert with VEGF. it may facilitate endothelial cell migration and proliferation. thus serving as a permissive | 1.42 | 3 |
| | | | angiogenic signal (By similarity) | | |
| **RIMS1** | RIMS1 | A0A3L7IGQ5 | spontaneous neurotransmitter secretion | 1.42 | 3 |
| **TECPR2** | TECPR2 | A0A3L7HLA9 | Propeller | 1.42 | 3 |
| **FAM98B** | FAM98B | A0A3L7HEW8 | Family with sequence similarity 98 member B | 1.39 | 3 |
| **CCNG2** | CCNG2 | A0A3L7INE3 | Cyclin. N-terminal domain | 1.39 | 3 |
| **PDE1C** | Phosphodiesterase | A0A3L7H700 | Calcium calmodulin-dependent 3'.5'-cyclic nucleotide phosphodiesterase 1C | 1.34 | 3 |
| **NA** | hypothetical protein | A0A061IHY9 | Arb2 domain | 1.32 | 3 |
| **CLEC2H** | CLEC2H (Fragment) | A0A3L7HAZ9 | C-type lectin domain family 2 member | 1.31 | 3 |
| **MUC19** | MUC19 | A0A3L7ICC8 | C-terminal cystine knot-like domain (CTCK) | 1.31 | 3 |
| **KIDINS220** | KIDINS220 | A0A3L7HD36 | KAP family P-loop domain | 1.30 | 3 |
| **BACH1** | BACH1 | A0A3L7HQE0 | Transcription regulator protein BACH2 | 1.28 | 3 |
| **NA** | hypothetical protein | NA | NA | 1.26 | 3 |
| **ZNF420** | ZNF420 (Fragment) | A0A3L7H5Z5 | C2H2-type zinc finger | 1.26 | 3 |
| **CHD7** | CHD7 | A0A3L7IBQ0 | chromodomain-helicase-DNA-binding protein 7 | 1.26 | 3 |
| **KLHL23** | KLHL23 | G3GU91 | Kelch-like protein 23 | 1.24 | 3 |
| **PCDHGC5** | PCDHGC5 | A0A3L7IA53 | Protocadherin | 1.24 | 3 |
| **DPP9** | DPP9 | A0A3L7HL01 | X-Pro dipeptidyl-peptidase (S15 family) | 1.14 | 2 |
| **METAP1** | Methionine aminopeptidase | A0A3L7ID05 | Metallopeptidase family M24 | 1.09 | 2 |
| **NFS1** | NFS1 | A0A3L7I997 | high mobility group | 1.09 | 2 |
| **AREL1** | AREL1 | A0A3L7HJW8 | E3 ubiquitin protein ligase 1 | 1.09 | 2 |
| **SLC24A1** | SLC24A1 (Fragment) | A0A3L7HTI0 | Sodium/calcium exchanger protein | 1.09 | 2 |
| **PSMD2** | 26S proteasome non-ATPase regulatory subunit 2 | A0A3L7HT01 | proteasome (prosome. macropain) 26S subunit. non-ATPase. 2 | 1.09 | 2 |
| **CgPICR_012677** | CXXC motif containing zinc binding protein (Fragment) | A0A3L7I411 | Eukaryotic protein of unknown function (DUF866) | 1.09 | 2 |
| **TOMT** | Catechol O-methyltransferase 2 | A0A061IIA7 | Transmembrane O-methyltransferase | 1.09 | 2 |
| **CgPICR_004773** | RBM26 (Fragment) | A0A3L7IF80 | RNA-binding protein 26 | 1.09 | 2 |
| **NPM2** | NPM2 | A0A3L7GQF2 | Nucleoplasmin/nucleophosmin domain | 1.09 | 2 |
| **OR5112** | OR51I2 | A0A3L7HXM0 | Olfactory receptor | 1.09 | 2 |
| **TRHR** | Thyroliberin receptor | A0A3L7HU01 | Belongs to the G-protein coupled receptor 1 family | 1.09 | 2 |
| **MAP3K7CL** | MAP3K7CL | A0A3L7HR29 | MAP3K7 C-terminal-like | 1.09 | 2 |
| **SLC7A14** | SLC7A14 | A0A3L7IHC7 | C-terminus of AA_permease | 1.09 | 2 |
| **NWD2** | NWD2 | A0A3L7IJ74 | Leucine-rich repeat and WD repeat-containing protein KIAA1239 | 1.09 | 2 |
| **TCP11L1** | TCP11L1 | A0A3L7HHG6 | T-complex protein 11 | 1.09 | 2 |
| **RPL23A** | RPL23A | A0A3L7GLT3 | ribosomal large subunit assembly | 1.09 | 2 |
| **PHF7** | PHF7 | A0A3L7GUY6 | PHD finger protein 7 | 1.09 | 2 |
| **CgPICR_022530** | Ig-like domain-containing protein | A0A3L7GYT4 | Immunoglobulin V-Type | 1.09 | 2 |
| **APOPT1** | APOPT1 | A0A3L7H873 | positive regulation of release of cytochrome c from mitochondria | 1.09 | 2 |
| **BRD9** | BRD9 | A0A3L7I9B2 | Bromodomain-containing protein 9 | 1.09 | 2 |
| **TCN2** | TCN2 | A0A3L7IR32 | cobalamin transport | 1.09 | 2 |
| **CgPICR_018207** | NCOA7 (Fragment) | A0A3L7I9U5 | Nuclear receptor coactivator | 1.09 | 2 |
| **ZNF764** | ZNF764 | A0A3L71IN1 | krueppel associated box | 1.09 | 2 |
| **RPS19** | 40S ribosomal protein S19 | A0A061HVT8 | ribosomal protein | 1.09 | 2 |
| **CgPICR_000061** | Mitogen-activated protein kinase (Fragment) | A0A3L7IAR0 | Mitogen-activated protein kinase 11 | 1.04 | 2 |
| **MED11** | Mediator of RNA polymerase II transcription subunit 11 | G3GYB2 | Mediator complex protein | 0.84 | 2 |
| **ST5** | ST5 | A0A3L7HUE8 | Suppression of tumorigenicity 5 | 0.84 | 2 |
| **DNAJB14** | DNAJB14 | A0A3L7IFA4 | Domain of unknown function (DUF1977) | 0.84 | 2 |
| **UNC80** | UNC80 (Fragment) | A0A3L7IBK8 | Cation channel complex component UNC80 | 0.84 | 2 |
| **NA** | hypothetical protein | A0A6P5R444 | secretion activator | 0.84 | 2 |
| **BRCC3** | Lys-63-specific deubiquitinase | A0A3L7H338 | histone H2A K63-linked deubiquitination | 0.84 | 2 |
| **SLC25A25** | SLC25A25 | A0A3L7HCH0 | Belongs to the mitochondrial carrier (TC 2.A.29) family | 0.84 | 2 |
| **CgPICR_002615** | Oxysterol-binding protein (Fragment) | A0A3L7IHX9 | Oxysterol-binding protein-related protein | 0.84 | 2 |
| **DDIT4** | DDIT4 | A0A3L7IM37 | DNA damage-inducible transcript 4 protein | 0.84 | 2 |
| **NA** | hypothetical protein | NA | NA | 0.84 | 2 |
| **TENM4** | TENM4 | A0A3L7HXF8 | GHH signature containing HNH/Endo VII superfamily nuclease toxin | 0.84 | 2 |
| **TSKS** | TSKS | A0A3L7HFV9 | serine kinase substrate | 0.84 | 2 |
| **ZNF865** | ZNF865 | A0A3L7H734 | Zinc finger protein | 0.84 | 2 |
| **CCDC34** | CCDC34 | G3I6E1 | coiled-coil domain-containing protein 34 | 0.84 | 2 |
| **WDR62** | WDR62 | A0A3L7H4L4 | WD domain. G-beta repeat | 0.84 | 2 |
| **MBD1** | MBD1 | A0A3L7IA99 | domain protein 1 | 0.84 | 2 |
| **PNPLA4** | PNPLA4 | A0A3L7GXY9 | Patatin-like phospholipase | 0.82 | 2 |
| **SCN1A** | Sodium channel protein | A0A3L7HGG0 | Mediates the voltage-dependent sodium ion permeability of excitable membranes | 0.78 | 2 |
| **CgPICR_013823** | Gamma-glutamyl carboxylase (Fragment) | A0A3L7H7E7 | gamma-glutamyl carboxylase activity | 0.76 | 2 |
| **UBE2Q1** | UBE2Q1 | A0A3L7IGGS | Ubiquitin-conjugating enzyme | 0.72 | 2 |
| **CgPICR_011804** | ALDH8A1 (Fragment) | A0A3L7I7C4 | Aldehyde dehydrogenase family | 0.72 | 2 |
| **HMGB1** | High mobility group protein 1 | A0A3L7HQA2 | HORMA domain | 0.71 | 2 |
| **SETBP1** | SETBP1 | A0A3L7ICU7 | SET-binding protein | 0.70 | 2 |
| **ICA** | Beta-1 metal-binding globulin | A0A3L7HNU8 | Transferrin | 0.70 | 2 |
| **SMAD1** | SMAD1 (Fragment) | A0A3L7GVH1 | embryonic pattern specification | 0.70 | 2 |
| **OR7G1** | Olfactory receptor | A0A3L7HT87 | Olfactory receptor 7G2-like isoform X2 | 0.68 | 2 |
| **NA** | hypothetical protein | A0A1U7QEJ4 | Partitioning defective 3 homolog B | 0.66 | 2 |
| **PRRT3** | PRRT3 | A0A3L7H7I2 | Proline-rich transmembrane protein 3 | 0.65 | 2 |
| **MID1IP1** | MID1IP1 | A0A3L7H1W2 | Mid1-interacting protein 1 | 0.64 | 2 |
| **ZNF462** | ZNF462 (Fragment) | A0A3L7I332 | Zinc finger protein 462 | 0.62 | 2 |
| **CRTC2** | CRTC2 | A0A3L7IGI5 | transcription coactivator 3 | 0.61 | 2 |
| **ENTPD7** | ENTPD7 | A0A3L7HY78 | GDA1/CD39 (nucleoside phosphatase) family | 0.61 | 2 |
| **CNNM3** | CNNM3 (Fragment) | A0A3L7INQ5 | CBS domain | 0.60 | 2 |
| **NA** | hypothetical protein | A0A6I9LQM5 | regulation of erythrocyte clearance | 0.60 | 2 |
| **PKP4** | PKP4 | A0A3L7HGM0 | Armadillo/beta-catenin-like repeat | 0.60 | 2 |
| **GRIA2** | Glutamate receptor | A0A3L7IFY0 | Eukaryotic homologues of bacterial periplasmic substrate binding proteins. | 0.60 | 2 |
| **NTSR2** | NTSR2 | A0A3L7HB91 | G-protein coupled receptor | 0.60 | 2 |
| **CCDC106** | CCDC106 | A0A3L7H5B0 | Coiled-coil domain-containing protein 106 | 0.59 | 2 |
| **RPS11** | 40S ribosomal protein S11 | G3I004 | rRNA binding | 0.59 | 2 |
| **NA** | hypothetical protein | NA | NA | 0.59 | 2 |
| **LMAN2L** | LMAN2L | A0A3L7INB6 | VIP36-like protein isoform X1 | 0.58 | 2 |
| **SERPIN12** | SERPINI2 | A0A3L7IJU3 | SERine Proteinase INhibitors | 0.58 | 2 |
| **GLB1L2** | Beta-galactosidase | A0A3L7HRR9 | Beta-galactosidase-1-like protein | 0.58 | 2 |
| **TLE3** | TLE3 | A0A3L7HNI8 | Transducin-like enhancer | 0.57 | 2 |
| **ENV** | ENV (Fragment) | A0A3L7ILG3 | ENV polyprotein (coat polyprotein) | 0.57 | 2 |
| **SPATA31A6** | SPATA31A6 | A0A3L7HVL5 | FAM75 family | 0.57 | 2 |
| **IGH-VJ558** | IGH-VJ558 | A0A3L7GXZ1 | Immunoglobulin V-Type | 0.57 | 2 |
| **GRAP** | GRAP | A0A3L7GMQ3 | GRB2-related adapter protein | 0.56 | 2 |
| **COL4A2** | COL4A2 | A0A3L7IPF5 | Collagen alpha-2(IV) chain | 0.55 | 2 |
| **ARL4D** | ADP-ribosylation factor-like protein 4D | G3INU1 | ADP-ribosylation factor-like | 0.38 | 6 |
| **CgPICR_006791** | Uncharacterized protein | A0A3L7IJM4 | T-cell receptor | 0.39 | 6 |
| **DEPTOR** | DEPTOR | A0A3L7IPG2 | Belongs to the universal ribosomal protein uL23 family | 0.39 | 6 |
| **DISP1** | DISP1 (Fragment) | A0A3L7HKM4 | Dispatched homolog 1 | 0.38 | 6 |
| **FXYD7** | FXYD domain-containing ion transport regulator | A0A3L7H4V0 | sodium channel regulator activity | 0.38 | 6 |
| **GRK6** | G protein-coupled receptor kinase | A0A3L7HWI8 | G protein-coupled receptor kinase | 0.39 | 6 |
| **MACF1** | MACF1 | A0A3L7I7Y9 | Microtubule-actin cross-linking factor 1 | 0.38 | 6 |
| **MORF4L2** | Mortality factor 4-like protein 2 | A0A3L7H233 | Mortality factor 4-like protein 2 | 0.39 | 6 |
| **PAG1** | PAG1 | A0A3L7I918 | Phosphoprotein associated with glycosphingolipid-enriched microdomains 1 | 0.38 | 6 |
| **RILPL1** | RILPL1 | A0A3L7HR56 | RILP-like protein 1 | 0.39 | 6 |
| **SIMC1** | SIMC1 | A0A3L7HVK6 | spliceosomal complex assembly | 0.38 | 6 |
| **STUB1** | STUB1 | A0A3L7HCC4 | STIP1 homology and | 0.38 | 6 |
| **SYTL1** | SYTL1 | A0A3L7I909 | Synaptotagmin-like protein 1 | 0.38 | 6 |
| **TMEM55A** | Phosphatidylinositol-4%2C5-bisphosphate 4-phosphatase | A0A3L7I3P7 | Transmembrane protein 55A | 0.39 | 6 |
| **TRIM2** | TRIM2 | A0A3L7IG82 | regulation of neuron apoptotic process | 0.38 | 6 |
| **VMN2R116** | VMN2R116 | A0A3L7H160 | 7 transmembrane sweet-taste receptor of 3 GCPR | 0.39 | 6 |
| **FOXO3** | FOXO3 | G3GXK7 | KIX-binding domain of forkhead box O. CR2 | 0.186 | 6 |
| **KDM5C** | KDM5A | G3H882 | PLU-1-like protein | 0.186 | 6 |
| **CTNNA1** | CTNNA3 | G3H9H8 | Catenin alpha-3 | 0.186 | 6 |
| **ARID1B** | ARID1B | G3HFW3 | SWI/SNF-like complex subunit BAF250/Osa | 0.257 | 6 |
| **LZTR1** | LZTR1 | G3HG01 | Leucine-zipper-like transcriptional regulator. 1 | 0.257 | 6 |
| **GPC3** | GPC3 | G3HGH9 | Cell surface proteoglycan that bears heparan sulfate | 0.186 | 6 |
| **TRAF7** | TRAF7 | G3HHB5 | E3 ubiquitin-protein ligase TRAF7 | 0.257 | 6 |
| **HNF1A** | HNF1A | G3HI63 | Hepatocyte nuclear factor 1-alpha | 0.257 | 6 |
| **PTPRK** | PTPRM | G3HIV1 | Protein tyrosine phosphatase. catalytic domain | 0.384 | 6 |
| **CTNNA1** | CTNNA3 | G3HN58 | Catenin alpha-3 | 0.186 | 6 |
| **GATA1** | GATA1 | G3HND3 | basophil differentiation | 0.257 | 6 |
| **RPL5** | RPL5 | G3HNJ6 | positive regulation of isoleucine-tRNA ligase activity | 0.257 | 6 |
| **NF2** | NF2 | G3HP12 | Ezrin/radixin/moesin family | 0.186 | 6 |
| **RPL5** | RPL5 | G3HVH8 | positive regulation of isoleucine-tRNA ligase activity | 0.384 | 6 |
| **SMARCD1** | SMARCD1 | G3HX65 | regulator of chromatin subfamily D member 1 | 0.186 | 6 |
| **EBF3** | EBF2 | G3HY76 | C2H2 zinc finger domain binding | 0.257 | 6 |
| **CDS1** | CDS1 | G3I5J8 | Phosphatidate cytidylyltransferase 1 | 0.257 | 6 |
| **CASP9** | CASP9 | G3I954 | Caspase recruitment domain | 0.257 | 6 |
| **ARHGAP35** | ARHGAP5 | G3ICT9 | rho GTPase-activating protein | 0.257 | 6 |
| **RXRA** | RXRA | G3IDY0 | Retinoic acid receptor RXR-alpha | 0.186 | 6 |
| **FAH** | FAH | A0A3L7HX59 | fumarylacetoacetase activity | 0.257 | 6 |

### Example 2: Optimized UPS by modified CHO cells with increased growth

Gene A encodes a negative regulator of the cell cycle, which slows the G1-to-S phase transition. By knocking out Gene A, it is anticipated that CHO cells will divide more rapidly and reach peak viable cell concentration (VCC) by Day 6 instead of Day 7 in a standard fed-batch bioprocess.

In future steps, CHO host cells will be transfected with Cas (e.g., Cas9) ribonucleoprotein complexes that include a guide RNA (sgRNA) specifically targeting Gene A. It is expected that at least 70% knockout efficiency will be achieved, confirmed through PCR-based detection of insertions or deletions, followed by Sanger sequencing and Inference of CRISPR Edits (ICE) analysis. Single-cell cloning will then be performed to isolate a fully characterized knockout clone lacking functional Gene A, which is predicted to exhibit a shorter doubling time than the parental line. The knockout clone will be used to generate recombinant production pools, with subsequent selection to develop pools or clones for biopharmaceutical manufacturing. These engineered cells are projected to reach peak VCC at least one day earlier than the wild-type line under comparable culture conditions. This anticipated reduction in process duration is expected to enhance overall efficiency without compromising specific productivity, providing a potential strategy for improving future manufacturing operations.

### Example 3: Predicted Growth Rate

Identified gene knockouts that significantly accelerate Chinese Hamster Ovary (CHO) cell growth were analysed using peak fold-change values to predict growth rate improvements. Individual gene knockouts yielded predicted growth improvements ranging approximately from 1% to 7% (FIG 11, left panel), calculated using the formula: ${Δ}_{growth} \left(%\right) = \left(\frac{{t}_{control}}{\frac{{t}_{total}}{\frac{{t}_{total}}{{t}_{control}} + {log}_{2} \left(FC+1\right)}}-1\right) * 100$

Assumptions made for this calculation is a 4 week cultivation time to generate the fold change in sgRNA counts and a linear constant improvement rate of genetic perturbations. To explore cumulative effects from combined genetic modifications, functionally coherent gene sets as listed in **Table 3** were derived from enriched knockout hits based on functional and pathway annotations, as listed in **Table 4.** The predicted growth improvements of individual genes within each curated set were summed to estimate cumulative effects. Predicted cumulative improvements for gene sets significantly surpassed individual knockout effects, reaching values ranging from 10 % up to almost 250% (FIG 11, right panel), corresponding to an effective cellular doubling time of approximately 6 hours in the fastest engineered CHO cell lines. Although this simplified additive model highlights the potential of combinatorial gene knockouts, actual biological interactions between genes may differ from this assumed additive effect.

## Claims

1. A modified Chinese Hamster Ovary (CHO) cell having an increased growth rate, wherein the cell comprises at least one modification in at least one endogenous coding sequence encoding at least one endogenous protein as defined in **Table 1,** preferably as defined in **Table 2,** wherein the at least one endogenous coding sequence is individually selected from the group of target genes as defined in **Table 6,** preferably as defined in **Table 7** or any homologue, orthologue, or paralogue thereof;
and wherein the at least one modification leads to the reduced transcription and/or reduced functional expression of said at least one endogenous protein, or of any homologue, orthologue, or paralogue thereof.

2. The modified CHO cell according to claim 1, wherein the at least one modification of at least one endogenous coding sequence is combined with at least one modification of at least one different endogenous coding sequence of a target gene selected from the group of HMGB1, STUB1, CAPN1, FOXO3, IFI16, KLHL22, LRRK2, PSAP, DCP1B, DDIT4, ARID1B, BRCC3, CCNG2, LATS2, NPM2, PML, SMARCD1, SWI5, GATA1, PSMD2, HORMAD2, PKP4, VEGFC, NDRG1, ANGPT2, BCAS3, MIA3, SPARC, MACF1, HLX, RALGAPA1, ARHGAP5, KIDINS220, CDH1, RILPL1, RIMS2, STK25, SLC12A2, ADAMTS15, MAPK11, PTBP1, SMAD1, ZEB1, CD24, CHD7, EXT1, JADE3, TF, EBF1, MED11, RXRA, TCOF1, FGF12, NF2, NCOA7, AKAP3, ME2, MPST, NADK2, PCK2, GPC3, PTPRM, GRAP, MBD1, RXRB, CASP9, CYP2C8, RPS19, HOXB3, NDRG1, FAH, LRTOMT, CDS1, OSBPL8, CEMIP, GLB1L2, CRTC3, SLC25A25, ALDH8A1, MID1IP1, HNF1A, OSTC, PANK4, GGCX, GTF2H1, SET, BACH2, ENTPD7, FCER1G, ESX1, MXD3, TBX15, GRIA2, AREL1, FBXO30, TRIM2, TLE1, TRIM2, UBE2Q1, RPL5, RPL7, RPS11, RPL23A, RPL35, SH3BGRL, SLC36A4, XPR1, ZDHHC20, MAPKBP1, TRAF7, EIF3E, BLOC1S6, CADPS, SYTL1, TPRG1L, SSH3, DISP1, GRK6, LZTR1, PAG1, SCN1A, FXYD7, TSPAN32, MAPKBP1, MAPKBP1, MAPKBP1, or of any homologue, orthologue, or paralogue thereof, preferably of a group of target genes from an individual panel as defined in **Table 3;** and the at least one combination of modifications leads to the reduced transcription and/or reduced functional expression of said at least one endogenous protein, or of any homologue, orthologue, or paralogue thereof.

3. The modified CHO cell according to claims 1 or 2, wherein said at least one endogenous protein has an amino acid sequence corresponding to SEQ ID NO: 1 to 235, or any homologue, orthologue, or paralogue thereof having an amino acid sequence having at least 75%, 76%, 77%, 78%, 79%, 80%, 81%, 82%; 83%, 84%, 85%, 86%, 87%, 88%, 89%, 90%, 91%, 92%, 93%, 94%, 95%, 96%, 97%, 98% or 99% sequence identity to a sequence corresponding to SEQ ID NO: 1 to 235, respectively.

4. The modified CHO cell according to any of the preceding claims, wherein the total concentration of the at least one modified endogenous protein is individually reduced by at least 20%, 25%, 30%, 35%, 40%, 45%, 50%, more preferably at least 55%, 60%, 65%, 70%, 75%, 80%, most preferably at least 80%, 81%, 82%, 83%, 84%, 85%, 86%, 87%, 88%, 89%, 90%, 91%, 92%, 93%, 94%, 95%, 96%, 97%, 98%, 99%, 100%, or even 100%, as determined by measuring the relative concentration of the at least one modified endogenous protein in ng/mL per sample obtained in a modified CHO cell in comparison to a reference sample obtained from a CHO wild-type cell not carrying the at least one modification in its genome.

5. The modified CHO cell according to any of the preceding claims, wherein said endogenous protein has a reduced transcription and/or reduced functional expression, wherein the modified CHO cell comprises at least one modification in at least three, at least four, at least five, at least six, at least seven, at least eight, at least nine, at least ten, at least eleven, at least twelve, at least thirteen, at least fourteen, at least fifteen, at least sixteen, at least seventeen, at least eighteen, at least nineteen, at least twenty or more different endogenous coding sequences encoding at least one endogenous protein and at least one recombinant sequence encoding at least one recombinant protein and/or at least one recombinant RNA.

6. The modified CHO cell according to any of the preceding claims, wherein said CHO cell comprises at least one recombinant gene encoding at least one recombinant protein and/or encoding at least one recombinant RNA molecule, optionally: wherein said at least one recombinant protein and/or at least one recombinant RNA molecule is a therapeutic molecule

7. The modified CHO cell according to any of the preceding claims, wherein said at least one modification is selected from the group consisting of at least one insertion, at least one deletions, and at least one substitution, including a base edit, or any combination thereof,
preferably wherein said at least one modification is present in exon 1 of the respective endogenous coding sequence
and/or
wherein said at least one modification in said endogenous sequence is a frame-shift mutation or a point mutation, the point mutation resulting in a stop codon.

8. The modified CHO cell according to any of the preceding claims, wherein said at least one modification in said at least one endogenous coding sequence allows for an optimized production of the at least one recombinant protein and/or at least one recombinant RNA,
(i) wherein said optimized production of the at least one recombinant protein and/or the at least one recombinant RNA is **characterized by** an increase
(a) in the yield of the at least one recombinant protein, and/or
(b) in the yield of the at least one RNA encoding said at least one recombinant protein cell, and/or
(c) in the yield of the at least one recombinant RNA,
in comparison to a reference sample obtained from a CHO wild-type cell not carrying the at least one modification in its genome, wherein the total concentration of the at least one modified endogenous protein is reduced by at least 20%, 25%, 30%, 35%, 40%, 45%, 50%, more preferably at least 55%, 60%, 65%, 70%, 75%, 80%, most preferably at least 80%, 81%, 82%, 83%, 84%, 85%, 86%, 87%, 88%, 89%, 90%, 91%, 92%, 93%, 94%, 95%, 96%, 97%, 98%, 99%, 100%, or even 100%, as determined by measuring the relative concentration of the at least one recombinant protein and/or at least one recombinant RNA in ng/mL per sample obtained in a modified CHO cell in comparison to a reference sample obtained from a CHO wild-type cell not carrying the at least one modification in its genome.

9. The modified CHO cell according to any of the preceding claims, wherein all alleles of said at least one endogenous coding sequence and/or regulatory sequence comprise at least one or more of said at least one modification.

10. The modified CHO cell according to any of the preceding claims, wherein the modified CHO cell comprises at least one modification in at least three, at least four, at least five, at least six, at least seven, at least eight, at least nine, at least ten, at least eleven, at least twelve, at least thirteen, at least fourteen, at least fifteen, at least sixteen, at least seventeen, at least eighteen, at least nineteen, at least twenty or more different endogenous coding sequences encoding at least one endogenous protein and at least one recombinant sequence encoding at least one recombinant protein and/or at least one recombinant RNA, wherein:
each endogenous coding sequence is selected from the group consisting of the following KEGG pathways: ribosome, spliceosome, cell cycle, nucleocytoplasmic transport, oxidative phosphorylation, biosynthesis of cofactors, mRNA surveillance pathway, nucleotide excision repair, RNA degradation, proteasome, DNA replication, basal transcription factors, RNA polymerase, homologous recombination and mismatch repair.

11. The modified CHO cell according to any one of the preceding claims, wherein the CHO cell undergoes a measurable and quantifiable enhancement of cell size (volume or mass) beyond that observed in a suitable reference or control population, preferably derived by using peak fold-change values to predict growth rate improvements.

12. A method for the production of at least one modified CHO cell according to any of the claims 1 to 11 comprising the steps:
(i) providing at least one CHO cell comprising at least one endogenous target nucleic acid segment;
(ii) providing at least one genome or transcriptome editing agent comprising at least one RNAi agent, or at least one site-directed endonuclease, preferably being selected from a meganuclease, a ZFN, a TALEN, a CRISPR-nuclease, or a nickase or nuclease-dead variant therefrom, or a nucleic acid molecule encoding the same, and optionally in case of a CRISPR-nuclease: providing at least one suitable, functional guide RNA molecule, or a nucleic acid molecule encoding the same;
(iii) introducing into said at least one CHO cell the at least one genome editing agent of step (ii);
(iv) obtaining at least one modified CHO cell comprising at least one modification in said at least one endogenous target nucleic acid segment according to step (i);
(v) optionally: selecting a further target nucleic acid segment and repeating steps (i) to (iv) at least one time to obtain at least one modified CHO cell comprising at least one further modification in a further endogenous target nucleic acid segment.

13. The method according to claim 12, wherein
(i) in step (ii) of claim 12 a CRISPR-nuclease is used; and
(ii) optionally: wherein a Cas enzyme is constitutively expressed by said at least one modified CHO cell; and
(iii) wherein the at least one suitable functional guide RNA molecule comprises a sequence corresponding to any of the nucleic acid sequences according to SEQ ID NOs: 1 to 1278 or corresponding to a nucleic acid sequence having at least 75%, 76%, 77%, 78%, 79%, 80%, 81%, 82%; 83%, 84%, 85%, 86%, 87%, 88%, 89%, 90%, 91%, 92%, 93%, 94%, 95%, 96%, 97%, 98% or 99% sequence identity to any one of the sequences according to SEQ ID NOs: 1 to 1278.

14. A method for the production of at least one recombinant molecule, preferably at least one recombinant protein and/or recombinant RNA, comprising the steps:
(a) providing at least one modified CHO cell according to any of the claims 1 to 11, wherein the at least one modified CHO cell comprises at least one recombinant gene encoding at least one recombinant protein, DNA or RNA;
(b) culturing said at least one target cell in a culture medium such that at least one recombinant molecule is transcribed and/or translated;
(c) harvesting said at least one recombinant molecule;
(d) purifying and/ or decontaminating said at least one recombinant molecule, preferably the at least one recombinant protein.

15. The use of at least one modified CHO cell according to any of the claims 1 to 11 for the production of at least one pharmaceutical drug.

16. A kit comprising
(a) at least one modified CHO cell according to any of claims 1 to 11, the modified CHO cell or the kit comprising:
(b.i) at least one nucleic acid molecule suitable for expressing at least one recombinant molecule of interest, preferably at least one recombinant protein of interest; optionally means for introducing the same into the modified CHO cell genome, or alternatively
(b.ii) alternatively at least one nucleic acid molecule encoding at least one recombinant molecule, preferably at least one recombinant protein; optionally means for introducing the same into the modified CHO cell genome; or
(b.iii) alternatively: at least one nucleic acid molecule suitable for transcribing at least one recombinant RNA molecule; optionally means for introducing the same into the modified CHO cell genome; or
(b.iv) alternatively: at least one nucleic acid molecule encoding at least one recombinant RNA molecule; optionally means for introducing the same into the modified CHO cell genome; and
(c) optionally: culture medium suitable for the at least one modified CHO cell according to step (a); and
(i) to replicate the at least one nucleic acid molecule according to any of the steps (b.i), (b.ii), (b.iii), and (b.iv) endogenously or exogenously; and
(ii.a) to express the at least one recombinant according to step (b.i) and/or (b.ii), and/or to transcribe the at least one recombinant RNA molecule according to step (b.iii) and/or (b.iv).
